# EUROPEAN PATENT SPECIFICATION

(11) **EP 2 609 140 B1**
(45) Date of publication and mention of the grant of the patent: **11.06.2014**
(21) Application number: 11750013.2
(22) Date of filing: 15.08.2011
(51) Int. Cl.: C08G 77/42

(54) **EMULSIONS CONTAINING SACCHARIDE SILOXANE COPOLYMER EMULSIFIERS AND METHODS FOR THEIR PREPARATION AND USE**
EMULSIONEN MIT SACCHARIDSILOXAN-COPOLYMER-EMULGATOREN SOWIE VERFAHREN ZU IHRER HERSTELLUNG UND VERWENDUNG
ÉMULSIONS CONTENANT DES ÉMULSIFIANTS À BASE DE COPOLYMÈRES DE SACCHARIDE-SILOXANES, PROCÉDÉS D'ÉLABORATION ET D'UTILISATION CORRESPONDANTS

(30) Priority: 23.08.2010 US 375938 P; 29.10.2010 US 407986 P
(43) Date of publication of application: 03.07.2013
(73) Proprietor: Dow Corning Corporation, Midland, Michigan 48686-0994 (US)
(72) Inventor: DELVALLE, Cindy, B-1180B Uccle (BE); JOFFRE, Eric, Jude, Midland MI 48642 (US); SCAVUZZO, Concettina, B-7160 Chap.-Lez-Herlaimont (BE); TOTH, Simon, Midland MI 48640 (US); VAN REETH, Isabelle, 201206, Shanghai (CN)
(74) Representative: Thomson, James B.
(86) International application number: PCT/US2011/047720
(87) International publication number: WO 2012/027145

(56) References cited:
- WO-A2-2006/127924
- US-A1- 2008 138 386

## Description

### CROSS-REFERENCE TO RELATED APPLICATIONS and STATEMENT REGARDING FEDERALLY SPONSORED RESEARCH

None.

### BACKGROUND OF THE INVENTION

Saccharide siloxanes are known in the art, for example, see WO2006/127924. Saccharide siloxanes comprising a hydroxyl functional saccharide component and an organosiloxane component were found to be useful when applied to hair, skin, fabric, paper, wood and other substrates. The saccharide component may be covalently bound to the organosiloxane at one or more pendant or terminal positions, or some combination thereof, through linkages including but not limited to ether, ester, and amide bonds.

### BRIEF SUMMARY OF THE INVENTION

A saccharide siloxane copolymer (copolymer) is useful as an emulsifier. Emulsions containing the copolymer are useful for personal care applications.

### DETAILED DESCRPTION OF THE INVENTION

A saccharide siloxane copolymer (copolymer) is useful as an emulsifier, for water in oil type emulsions, such as water in silicone emulsions. The copolymer comprises a saccharide component and a siloxane component. The siloxane component forms the backbone of the copolymer molecule. Saccharide components may be bonded to the siloxane backbone in terminal groups, pendant groups, or both terminal and pendant groups. Alternatively, the saccharide component may be bonded to the siloxane backbone in a pendant group.

The copolymer may be a solid or a fluid under ambient conditions of temperature and pressure, *e.g*., at 25 °C and 101 kPa (760 mmHg). Whether the copolymer is a solid at ambient conditions, or a fluid such as a liquid or a gum, depends on various factors including the degree of polymerization (DP) of the copolymer. The copolymer may have a DP ranging from 2 to 15,000, alternatively 50 to 5,000, alternatively, 100 to 1,000, alternatively 50 to 1,000, and alternatively 100 to 400.

Alternatively, the copolymer may be a fluid under ambient conditions. The viscosity of the copolymer depends on various factors including the degree of polymerization (DP) of the copolymer. The copolymer may have a DP ranging from 2 to 500, alternatively 5 to 500, alternatively, 25 to 500, alternatively 50 to 400, alternatively100 to 400, and alternatively 50 to 350.

### Copolymer

The copolymer may have unit formula (I):
(R¹₍₃₋ₐ₎R²ₐSiO_{1/2})_{b} (R¹_{(2-c)}R²_{c}SiO_{2/2})_{d} (R²SiO_{3/2})ₑ (R¹₃SiO_{1/2})_{f} (R¹₂SiO_{2/2})_{g} (R¹SiO_{3/2})ₕ (SiO_{4/2})ᵢ. Each subscript a is independently 0, 1, 2, or 3. Subscript c is 0, 1, or 2. On average per molecule, the quantity (a + c) may be at least 1. Subscript b is 0 or greater. Subscript d is 0 or greater. Subscript e is 0 or greater. The quantity (b + d + e) is 1 or greater. Subscript f is 0 or greater. Subscript g is 0 or greater. Subscript h is 0 or greater. Subscript i is 0 or greater. When e, h, and i are all 0, then the saccharide-siloxane polymer is linear, and the polymer may have the following formula (II):

R²ₐR¹₍₃₋ₐ₎SiO-[(R²R¹SiO)ⱼ (R¹₂SiO)ₖ]ₘ-SiR¹₍₃₋ₐ₎ R²ₐ.

In the formulae above, each R¹ can be the same or different. Each R¹ comprises hydrogen, an alkyl group of 1 to 12 carbon atoms, an organic group, or a group of formula R³-Q. Q comprises an epoxy, cycloalkylepoxy, primary or secondary amino, ethylenediamine, carboxy, halogen, vinyl, allyl, anhydride, or mercapto functionality.

Subscripts j and k are integers ranging from 0 to 15,000 and may be the same or different. Alternatively, each subscript j may be 0 to 500 and each subscript k may be 0 to 500. Each subscript a is independently 0, 1, 2, or 3. Alternatively, each subscript a may be 0. When subscript a is 0, then at least one of subscripts j and k is greater than 0, and all of the saccharide components are in pendant groups (not terminal groups) on the copolymer. Subscript m is an integer such that the copolymer has a molecular weight less than 1 million. Subscripts y, m, and n may have values such that the copolymer is a fluid under ambient conditions. Subscript y, and at least one of subscripts m and n, may be greater than 0 such that a saccharide component is in a pendant group on the copolymer.

Each R² has the formula Z-(G¹)ₙ-(G²)ₒ, and there is an average of at least one R² per polymer molecule. G¹ is a saccharide component comprising 5 to 12 carbon atoms. The quantity (n + o) has a value ranging from 1 to 10, and subscript n or subscript o can be 0. G² is a saccharide component comprising 5 to 12 carbon atoms, and G² is additionally substituted with organic or organosilicon radicals. Z is a linking group. Each Z is independently selected from the group consisting of:

-R³-NHC(O)-R⁴-;

-R³-NHC(O)O-R⁴-;

-R³-NH-C(O)-NH-R⁴-;

-R³-C(O)-O-R⁴-;

-R³-O-R⁴-;

-R³-CH(OH)-CH₂-O-R⁴-;

-R³-S-R⁴-;

-R³-CH(OH)-CH₂-NH-R⁴-;

-R³-N(R¹)-R⁴-;

-NHC(O)-R⁴-;

-NHC(O)O- R⁴-;

-NH-C(O)-NH-R -;

-C(O)-O-R⁴-;

-O-R⁴-;

=CH(OH)=CH₂-O=R⁴-;

-S-R⁴-;

-CH(OH)-CH₂-NH-R⁴-;

-N(R¹)-R⁴-;

-R³-NHC(O)-;

-R³-NHC(O)O-;

-R³-NH-C(O)-NH-;

-R³-C(O)-O-;

-R³-O-;

-R³-CH(OH)-CH₂-O-;

-R³-S-;

-R³-CH(OH)-CH₂-NH-; and

-R³-N(R¹)-.

Each R³ and each R⁴ is independently a divalent spacer comprising a group of formula (R⁵)ᵣ(R⁶)ₛ(R⁷)ₜ, where at least one of subscripts r, s and t is 1. Each R⁵ and each R⁷ are independently either an alkylene group of 1 to 12 carbon atoms or a group of formula (R⁹O)ₚ, where R⁹ is a divalent organic group such as an alkylene group of 1 to 12 carbon atoms, subscript p is an integer ranging from 1 to 50, and each R⁹O may be the same or different. Alternatively, each R⁵ and each R⁷ are independently an alkylene group of 1 to 12 carbon atoms, and the copolymer may be free of groups of formula R⁹O. Without wishing to be bound by theory, it is thought that copolymers free of groups of formula R⁹O when used as emulsifiers may provide low odor emulsions. R⁶ is -N(R⁸)-, where R⁸ is hydrogen, an alkyl group of 1 to 12 carbon atoms, a group of formula Z-X where Z is previously defined, or R³. Each X is independently a divalent carboxylic acid, phosphate, sulfate, sulfonate or quaternary ammonium radical. The saccharide-siloxane polymer is a reaction product of a functionalized organosiloxane polymer and at least one hydroxy-functional saccharide such that the organosiloxane component is covalently linked via the linking group, Z, to the saccharide component.

Alternatively, the saccharide-siloxane polymer may have the formula (III): 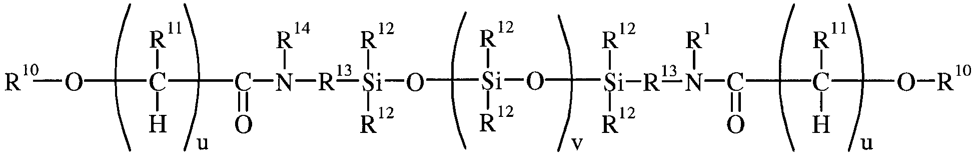 where each subscript u is independently 5 to 12 and subscript v has a value ranging from 0 to 10,000, alternatively 11 to 300.

Each R¹⁴ is independently a hydrogen atom or a monovalent hydrocarbon group of 1 to 4 carbon atoms. Each R¹³ is independently a divalent organic group. Divalent organic groups are exemplified by unsubstituted divalent hydrocarbon groups, e.g., alkylene groups such as ethylene, propylene, and butylene and substituted divalent hydrocarbon groups such as divalent amino-functional groups such as propylaminoethyl (e.g., -(CH₃)₃N-(CH₂)₂-). Alternatively, each R¹³ may be propyl. Alternatively, each R¹³ may be propylaminoethyl.

Each R¹² is independently a monovalent unsubstituted hydrocarbon group. Examples of monovalent unsubstituted hydrocarbon groups include alkyl groups, alkenyl groups, cycloalkyl groups, and aromatic groups. Alkyl groups include methyl, ethyl, and propyl. Alkenyl groups include vinyl and allyl. Cycloalkyl groups include cyclopentyl and cyclohexyl. Aromatic groups include phenyl, tolyl, xylyl, and benzyl.

Each R¹¹ is independently a hydrogen atom, a hydroxyl group, an alkoxy group, or a saccharide group. Alkoxy groups are exemplified by methoxy, ethoxy, propoxy, and butoxy.

Each R¹⁰ is a hydrogen atom or a monovalent substituted or unsubstituted hydrocarbon group.

Examples of the saccharide-siloxane polymer with this formula include the following.

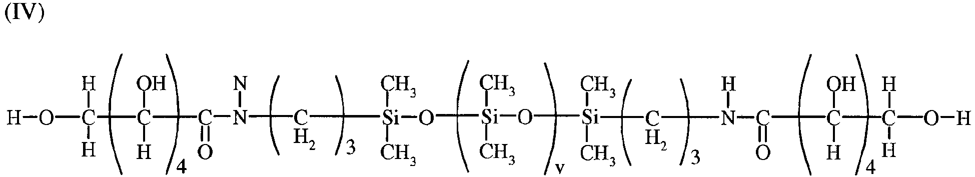

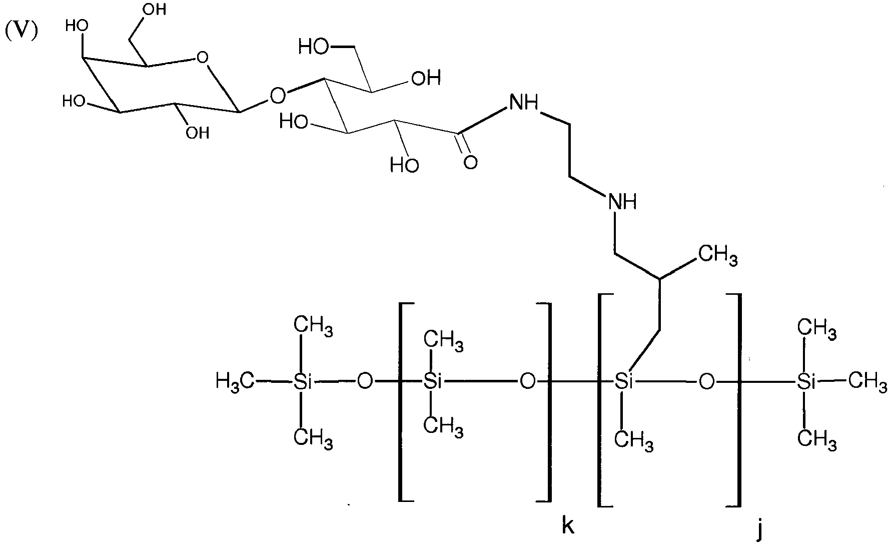

The copolymers of formulae (I) to (V) may be prepared by a method comprising:
1) reacting an organofunctional polyorganosiloxane with a sugar moiety to produce a saccharide siloxane copolymer as described above in the presence of a solvent, 2) adding an oil, and 3) removing all or a portion of a solvent.

In one embodiment, the method for making the copolymer comprises:
1) reacting (A) an amino-functional polyorganosiloxane and (B) a lactone in the presence of (C) a solvent, 2) adding (D) an oil, and thereafter 3) removing all or a portion of ingredient (C). The oil can be added in the method any time before removing all or a portion of the solvent. Ingredient (A) is an amino-functional polyorganosiloxane. Ingredient (A) may have the formula (VI): 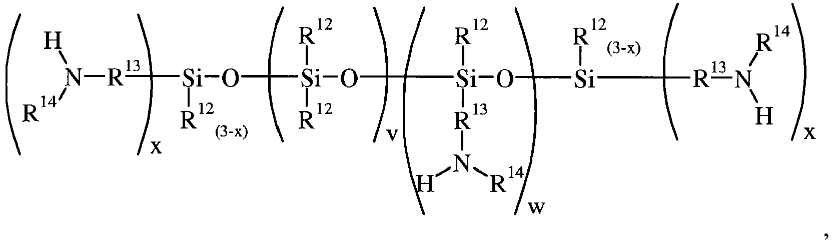 where
   each R¹² is independently a monovalent hydrocarbon group;
   each R¹³ is independently a divalent organic group;
   each R¹⁴ is independently a hydrogen atom or a monovalent hydrocarbon group of 1 to 4 carbon atoms;
   each subscript x is independently 0 or 1;
   subscript v has a value ranging from 0 to 15,000; and
   subscript w has a value ranging from 0 to 15,000.

Ingredient (A) is exemplified by trimethylsiloxy-terminated poly(dimethylsiloxane/methyl(aminoethylaminoisobutyl)siloxane), trimethylsiloxy-terminated poly(dimethylsiloxane/methyl(aminopropyl)siloxane), trimethylsiloxy-terminated poly(dimethylsiloxane/methyl(aminoethylaminopropyl)siloxane), and combinations thereof.

Ingredient (B) is a lactone. Ingredient (B) may have formula (VII): 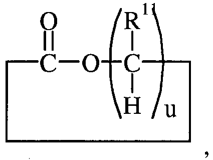 where R¹¹ and subscript u are as described above. Ingredient (B) is exemplified by butyrolactone, epsilon caprolactone and delta gluconolactone. Alternatively, ingredient (B) may be lactobionolactone.

Ingredient (C) may be any solvent in which the lactone is miscible. Ingredient (C) may be an alcohol. Suitable alcohols for ingredient (C) include methanol, ethanol, n-propanol, isopropanol, 2-propanol, isobutanol, n-butanol, and combinations thereof.

Ingredient (D) is an oil. The oil may be a silicone oil such as a polydialkylsiloxane having a viscosity of 1 to 350 cSt. Such silicone oils are commercially available as DOW CORNING® 200 Fluids (with viscosities ranging from 2 centiStokes 350 centiStokes), and DOW CORNING® FZ-3196, DOW CORNING® 244 Fluid, and DOW CORNING@ 245 Fluid from Dow Corning Corporation of Midland, Michigan, U.S.A. Dimethicone oils from Dow Corning Corporation include 244 Fluid, 245 Fluid, and 200 Fluids with viscosity of 2 cSt, 5 cSt, 10 cSt 20 cSt, 50 cSt, 100 cSt, or 350 cSt.

Alternatively, certain organic oils are suitable for use in the emulsion. Suitable organic oils include esters, vegetable and/or mineral oils, hydrocarbon oils, or fatty alcohols.

Suitable esters include isopropyl myristate, octyl octanonanoate, decyl oleate, isopropyl palmitate, glyceryl stearate, ethylhexyl stearate, isopropyl isostearate, C12-C15 alkyl benzoate, octyl cocoate, octyl palmitate, myristyl lactate, and dioctyl adipate. Examples of esters further comprise cetyl ethylhexanoate (which is commercially available as Schercemol™ CO Ester from The Lubrizol Corporation of Wickliffe, Ohio, U.S.A.) and triethylhexanoin (which is commercially available as Schercemol™ GTO Ester, also from Lubrizol).

Suitable vegetable and mineral oils include almond oil, apricot kernel, avocado oil, castor oil, evening primrose, jojoba oil, sunflower oil, olive oil, wheat germ oil, and mineral oil.

Suitable hyrocarbon oils include petrolatum, mineral oil, squalene, capric/caprylic triglyceride; an alkane of at least 12 carbon atoms. For example, long chain alkanes (e.g., alkanes having at least 12 carbon atoms, such as isododecane or isohexadecane) may be used as the organic oil.

Suitable fatty alcohols that include strearyl alcohol, cetyl alcohol, and combinations thereof.

Alternatively, the copolymer may be prepared by a method comprising reacting an epoxy functional polyorganosiloxane with an n-alkyl glucamine such as n-methyl glucamine. The epoxy functional polyorganosiloxane may be prepared by methods known in the art, such as by hydrosilylation of ingredients comprising an alkenyl functional epoxy containing compound and a polyorganohydrogensiloxane. The alkenyl functional epoxy containing compound may be allyl glycidyl ether, dodecenyl glycidyl ether, tetradecenyl glycidyl ether, or octadecenylglycidyl ether. The ingredients may optionally further comprise further comprise an alkene, such as undecene. Alternatively, one skilled in the art could react the n-alkyl-glucamine first with the alkenyl functional epoxy containing compound and thereafter perform the hydrosilylation reaction to attach the product thereof to the polyorganohydrogensiloxane.

Alternatively, the copolymer may be prepared by a method comprising:
1) reacting an n-alkyl-glucamine with an alkenyl functional epoxy compound, and
2) hydrosilylating the product of step 1) with a polyorganohydrogensiloxane.

Steps 1) and 2) may be performed sequentially. Alternatively, step 1 and step 2 may be combined and performed simultaneously.

In this method, the alkenyl functional epoxy containing compound may be allyl glycidyl ether, dodecyl glycidyl ether, tetradecyl glycidyl ether, or octadecylglycidyl ether. The n-alkyl glucamine may be n-methyl glucamine.

The methods described above may be performed neat or in the presence of a solvent. The solvent may be an alcohol such as methanol, ethanol, propanol, butanol, or a combination thereof. Alternatively, the organo-functional polyorganosiloxane (e.g., amine functional polyorganosiloxane, or epoxy functional polyorganosiloxane, or the polyorganohydrogensiloxane) may be dissolved in a solvent such as ethanol with the other ingredients used in the method. All or a portion of the solvent may be removed, for example, by stripping or distillation, after the method is complete. Alternatively, the copolymer may be left in the solvent after the method is complete, for example, if the solvent is a suitable ingredient for an emulsion in which the copolymer will be formulated.

The copolymer is combined with an oil in the methods described above. The oil may be added in addition to the solvent. The oil may be added before reacting the ingredients to make the copolymer. Alternatively, the oil may be added during and/or after making the copolymer and before removal of any solvent. Alternatively, the oil may be added after a portion of the solvent is removed. Alternatively, the oil may be added after all of the solvent is removed.

The methods described above may be performed by heating. The exact temperature depends on various factors including the specific ingredients selected, however, temperature may range from 50 °C to 100 °C and reaction time for each step may be several hours, alternatively, up to 10 hours, alternatively 1 to 10 hours. The first and second steps in the methods described above may be performed sequentially. Alternatively, step 1 and step 2 may be combined and performed simultaneously.

In the methods described above a molar excess may be used of the functionality on the reagent reacting with the functionality on the polyorganosiloxane. For example, in the hydrosilylation of allyl glycidyl ether with an SiH intermediate polyorganosiloxane, a 1.1:1 ratio is used of the moles allyl glycidyl ether to the moles of SiH. The ratio for the reagent to siloxane bonded functionality may be as large as 1.8:1. Alternatively, the molar ratio may range from 1:1 to 1.8:1, alternatively 1.1:1 to 1.5:1.

Alternatively, the molar ratio of sugar lactone to amine may be 1:1, calculated from amine value of the amine functional polyorganosiloxanes. However, the molar ratio of sugar functionality in the sugar lactone to amine in the amine functional polyorganosiloxane may range from 0.5:1 to 2.0:1.

Adding the oil may be performed by any convenient means, such as mixing. Removing all or a portion of the solvent may be performed by any convenient means, such as stripping or distillation. The product of step 2) may be heated and/or exposed to reduced pressure to facilitate stripping and/or distillation.

Alternatively, in a specific embodiment, 100 parts of the organofunctional polyorganosiloxane, 15 to 200 parts of the solvent and a stochiometric amount of sugar moiety are combined and heated to a temperature ranging from 40 °C to 74 °C while being continuously stirred. The reaction is carried out until all sugars are attached to the polyorganosiloxane chain. At this point the oil is added and well mixed. The copolymer and oil are present in an amounts such that the weight ratio of copolymer/oil ranges from 1/1 to 1/50, alternatively 1/4 to 1/20, and alternatively 1/8 to 1/10. In the next step the solvent is stripped off at a temperature up to 74 °C and at up to full vacuum.

In an alternative specific embodiment of the method, 100 parts of organofunctional polyorganosiloxane, 15 to 200 parts of the solvent and a stoichiometric amount of sugar moiety are mixed. The mixture is heated to up to 74 °C while being continuously stirred. The reaction is carried out until all sugars are attached to polyorganosiloxane chain. At this point the solvent may be partially stripped off, *e.g*., from less than 200 parts down to 15 parts, and thereafter the oil added to the mixture and mixed. The copolymer and oil are present in amounts such that the weight ratio of copolymer/oil ranges from 1/1 to 1/50, alternatively 1/4 to 1/20, and alternatively 1/8 to 1/10. In the next step, the solvent may be stripped off by heating at a temperature at up to 74 °C and at up to full vacuum.

### Method of Making Emulsion

The emulsion may be prepared by a method comprising adding the aqueous phase to the oil phase comprising the copolymer and oil combination described above. The aqueous phase may be added to the oil phase in increments with mixing between additions. The resulting combination of aqueous and oil phases may be subjected to high shear. The oil forms the external or continuous phase. Mixing may be performed, for example, by mixing with a cross stirrer at 700 to 1,000 revolutions per minute (rpm) while adding the aqueous phase. After the aqueous phase has been added, the resulting mixture may optionally be further mixed at 1,000 to 2,000 rpm for a period of time such as 1 second to 10 minutes, alternatively 1 minute to 5 minutes. For example, mixing conditions after all the aqueous phase have been added may include mixing for 1 minute at 1,000 rpm and then 5 minutes at 2,000 rpm.

The high shear mixing may be performed using special equipment, which allows to the emulsion mix at very high shear to reduce particle size and increase the viscosity of the emulsion. High shear mixing may improve stability of the emulsion. The high shear mixing may be performed with a commercially available high shear device, *e.g.*, a homogenizer such as a T25 Digital ULTRA-TURRAX® commercially available from IKA of Wilmington, North Carolina, U.S.A. or a homogenizer such as L4RT commercially available from Silverson Machines Ltd. of England. The exact conditions for high shear mixing will vary depending on factors such as the initial viscosity of the emulsion, however, high shear conditions are exemplified by mixing the emulsion at 7,000 to 8,000 rpm for 1 second to 1 minute, alternatively 15 seconds.

It will be understood by one of ordinary skill in the art that there is a continuum for the ease with which a desired emulsion forms. The emulsions described herein share similar constraints with other emulsions. That is, they are thermodynamically unstable and need an input of energy to initiate emulsification. Simple agitation via mixing may be sufficient, or higher shear means including the employment of high shear devices may be used. Alternatively, an inversion method may be used.

A degree of agitation necessary to form the emulsion may require employment of mixing devices. Mixing devices typically provide the required energy input. Non-limiting examples of these mixing devices spanning the shear range include: 1) a vessel with an impeller, for example, propeller, pitched blade impeller, straight blade impeller, Rushton impeller, or Cowles blade; 2) kneading type mixers, for example, Baker-Perkins; 3) high shear devices which use positive displacement through an orifice to generate shear, for example, homogenizer, sonolator, or microfluidizer; 4) high shear devices using a rotor and stator configuration, for example, colloid mills, homomic line mills, homogenizers from IKA, or Bematek; 5) continuous compounders with single or dual screws; 6) change can mixers with internal impellers or rotor/stator devices, for example, Turello mixer; and 7) centrifugal mixers, for example, Hauschild speedmixers. Combinations of mixing devices can also provide benefits, for example a vessel with an impeller can be connected to a high shear device. High shear devices are known in the art and are commercially available, for example, the high shear device may be a homogenizer such as a T25 Digital ULTRA-TURRAX® commercially available from IKA of Wilmington, North Carolina, U.S.A. or high shear mixer from Silverson Machines Ltd. of England.

The choice of mixing device is based on the type of internal phase to be emulsified. For example, low viscosity internal phases can be emulsified using high shear devices which use positive displacement through an orifice. However, in the case of high viscosity internal phases, a rotor/stator device, twin screw compounder or change can mixer are often better choices.

In a specific embodiment, water in oil emulsion samples were prepared according to the following general procedure. The oil phase was prepared by mixing an emulsifier with an oil. The oil was isopropyl myristate or DOW CORNING ® 200 Fluid, a silicone oil with a viscosity of 5 cSt, which is commercially available from Dow Corning Corporation. The emulsifier was a copolymer as described above or a comparative emulsifier. In each 20 gram (g) sample of oil phase, the oil phase contained 2 g emulsifier and 18 g oil.

The aqueous phase was prepared by mixing water and sodium chloride in a water:NaCl weight ratio ranging from of 39:1 to 99:1. For each sample, 80 g of aqueous phase was prepared.

For each sample, the aqueous phase was added to the oil phase in 5 g increments. Between the addition of each increment, the sample was mixed for 40 seconds (s) at 3400 revolutions per minute (rpm) in a DAC150 FlackTek^{™} SpeedMixer^{™} (commercially available from FlackTek, Inc. of Landrum, South Carolina, U.S.A.) to provide a coarse emulsion.

After all the aqueous phase was added, the resulting coarse emulsion was subjected to shear at ≥7,000 rpm in a homogenizer (T25 Digital ULTRA-TURRAX® commercially available from IKA of Wilmington, North Carolina, U.S.A.) to provide the final emulsion sample.

The aqueous phase may be present in an amount ranging from 20% to 95%, preferably 40 to 90%, and most preferably 60 % to 80 % by weight based on the weight of the emulsion.

### Personal Care Applications

The emulsion described above is useful in personal care applications. When the emulsion described above is used in personal care applications, the emulsion may further comprise an additional ingredient, such as those described above. The additional ingredient may be selected from additional silicones, aerosols, anti-oxidants, cleansing agents, colorants, additional conditioning agents, deposition agents, electrolytes, emollients and oils, exfoliating agents, foam boosters, fragrances, humectants, occlusive agents, pediculicides, pH control agents, pigments, preservatives, biocides, other solvents, stabilizers, sunscreening agents, suspending agents, tanning agents, other surfactants, thickeners, vitamins, botanicals, waxes, rheology-modifying agents, antiperspirants, anti-dandruff, anti-acne, anti-carie and wound healing-promotion agents, an additional oil, a hydrophilic medium, a filler, a fiber, a film forming polymer, an additional surfactant and/or emulsifier, a dyestuff, a structuring agent, an active ingredient, a fragrance, a preservative, and combinations thereof. Alternatively, the additional ingredient can be selected from an additional oil, a hydrophilic medium, a filler, a fiber, a film forming polymer, an additional surfactant and/or emulsifier, a dyestuff, a structuring agent, an active ingredient (such as a personal care active), a fragrance, a preservative, or a combination thereof.

### Additional Oil

The additional oil may be another oil selected from the oils as described above, or the oil may be chosen from hydrocarbon-based oils, silicone oils and fluorinated oils. The oil may be chosen from volatile oils and non volatile oils, and mixtures thereof.

For purposes of this application, the term "hydrocarbon-based oil" means an oil formed essentially from, or even consisting of, carbon and hydrogen atoms, and possibly oxygen and nitrogen atoms, and containing no silicon or fluorine atoms; it may contain ester, ether, amine, or amide groups.

For purposes of this application, the term "silicone oil" means an oil containing at least one silicon atom, and alternatively containing ≡Si-O- groups.

For purposes of this application, the term "fluorinated oil" means an oil containing at least one fluorine atom.

For purposes of this application, the term "volatile oil" means an oil (or non-aqueous medium) capable of evaporating on contact with the skin in less than one hour, at room temperature and atmospheric pressure. The volatile oil may be a volatile cosmetic oil, which is liquid at room temperature, especially having a non-zero vapor pressure, at room temperature and atmospheric pressure, in particular having a vapor pressure ranging from 0.13 Pa to 40 000 Pa (10 -3 to 300 mmHg), alternatively ranging from 1.3 Pa to 13 000 Pa (0.01 to 100 mmHg) and alternatively ranging from 1.3 Pa to 1300 Pa (0.01 to 10 mmHg).

In addition, the volatile oil generally has a boiling point, measured at atmospheric pressure, ranging from 150 °C to 260 °C and alternatively ranging from 170 °C to 250 °C .

The emulsion may comprise a volatile hydrocarbon-based oil chosen especially from hydrocarbon-based oils with a flash point ranging from 40 °C to 102 °C, alternatively ranging from 40 °C to 55 °C and alternatively ranging from 40 °C to 50 °C .

The volatile oil may be present in the emulsion in an amount ranging from 0.1 % to 80 % by weight, alternatively ranging from 1 % to 70 % by weight, and alternatively ranging from 5 % to 50 % by weight, relative to the total weight of the emulsion.

The emulsion may comprise at least one non-volatile oil in a non-volatile liquid fatty phase. The non-volatile oil may be present in an amount ranging from 0.1 % to 80 % by weight, alternatively ranging from 0.5 % to 60 % by weight, and alternatively ranging from 1 % to 50 % by weight relative to the total weight of the non-volatile liquid fatty phase.

The volatile hydrocarbon-based oils may be selected from hydrocarbon-based oils containing from 8 to 16 carbon atoms, and especially branched C8-C16 alkanes, for instance C8-C16 isoalkanes of petroleum origin (also known as isoparaffins), for instance isododecane (also known as 2 ,2 , 4 , 4 , 6-pentamethylheptane), isodecane and isohexadecane, for example the oils sold under the trade names Isopar or Permethyl, branched C8-C16 esters and isohexyl neopentanoate, and combinations thereof.

Volatile oils that may also be used include volatile silicones, for instance volatile linear or cyclic silicone oils, especially those with a viscosity ≤ 8 centistokes (8 x 10⁶ m²/s) and especially containing from 2 to 7 silicon atoms, these silicones optionally comprising alkyl or alkoxy groups containing from 1 to 10 carbon atoms.

Volatile fluorinated solvents such as nonafluoro-methoxybutane or perfluoromethylcyclopentane are also suitable for use in the composition.

Non-volatile hydrocarbon-based oils include, but are not limited to, hydrocarbon-based oils of plant origin, such as triesters of fatty acids and of glycerol, the fatty acids of which may have varied chain lengths from 4 to 24 carbon atoms, these chains possibly being linear or branched, and saturated or unsaturated. These oils are exemplified by wheat germ oil, sunflower oil, grapeseed oil, sesame seed oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppyseed oil, pumpkin oil, marrow oil, blackcurrant oil, evening primrose oil, millet oil, barley oil, quinoa oil, rye oil, safflower oil, candlenut oil, passionflower oil or musk rose oil; or caprylic and/or capric acid triglycerides; synthetic ethers containing from 10 to 40 carbon atoms; apolar hydrocarbon-based oils, for instance squalene, linear or branched hydrocarbons such as liquid paraffin, liquid petroleum jelly and naphthalene oil, hydrogenated or partially hydrogenated polyisobutene, isoeicosane, squalane, decene/butene copolymers and polybutene/polyisobutene copolymers, and polydecenes, and mixtures thereof; synthetic esters, for instance oils of formula R'COOR" in which R' represents a linear or branched fatty acid residue containing from 1 to 40 carbon atoms and R" represents a hydrocarbon-based chain, which is especially branched, containing from 1 to 40 carbon atoms, on condition that R'+ R" > 10, for instance cetostearyl octanoate, isopropyl myristate, isopropyl palmitate, alkyl benzoates of 12 to 15 carbon atoms, hexyl laurate, diisopropyl adipate, isononyl isononanoate, 2-ethylhexyl palmitate, isostearyl isostearate, alcohol or polyalcohol octanoates, decanoates or ricinoleates, for instance propylene glycol dioctanoate; hydroxylated esters, for instance isostearyl lactate or diisostearyl malate; and pentaerythritol esters; fatty alcohols that are liquid at room temperature with a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpenta- decanol; higher fatty acids such as oleic acid, linoleic acid or linolenic acid; carbonates; acetates; citrates; and combinations thereof.

The non-volatile silicone oils may be: non-volatile polydimethylsiloxanes (PDMS); polydimethylsiloxanes comprising alkyl or alkoxy groups, which are pendent and/or at the end of a silicone chain, these groups each containing from 3 to 40 carbon atoms; phenylsilicones; optionally fluorinated polyalkylmethylsiloxanes; polysiloxanes modified with fatty acids, fatty alcohols or polyoxyalkylenes, and combinations thereof. Alkylmethylsiloxanes, which generally will have the formula Me₃SiO[Me₂SiO]_{A}[MeR'"SiO]_{B}SiMe₃, in which R'" is a hydrocarbon group containing 6 to 30 carbon atoms, Me represents methyl, and the degree of polymerization (DP), *i*.*e*., the sum of A and B ranges from 3 to 50. Both the volatile and liquid species of alkymethysiloxanes can be used in the composition.

The oil may alternatively comprise a silicone carbinol. These materials are described in WO 03/101412 A2, and can be commonly described as substituted hydrocarbyl functional siloxane fluids or resins.

The emulsion may contain an oil with a molar mass ranging from 650 to 10,000 g/mol, which may be selected from: lipophilic polymers such as polybutylenes; polyisobutylenes, for example hydrogenated polyisobutylenes; polydecenes and hydrogenated polydecenes; vinylpyrrolidone copolymers such as a vinylpyrrolidone/1-hexadecene copolymer (MM = 7300 g/mol); esters such as linear fatty acid esters with a total carbon number ranging from 35 to 70, for instance pentaerythrityl tetrapelargonate; hydroxylated esters such as polyglyceryl-2 triisostearate; aromatic esters such as tridecyl trimellitate; and pentaerythritol esters, and triisoarachidyl citrate, pentaerythrityl tetraisononanoate, glyceryl triisostearate, glyceryl tris (2-decyl) tetradecanoate, pentaerythrityl tetraisostearate, polyglyceryl-2 tetraisostearate, and combinations thereof.

The emulsion may comprise a fluid silicone compound such as a silicone gum or a silicone oil of high viscosity.

A polydimethylsiloxane with a viscosity at 25 °C ranging from 10 to 10,000,000 cSt., alternatively 1,000 to 2,500,000 cSt., alternatively 5,000 to 1,000,000 cSt., and alternatively 10,000 to 60,000 cSt. may be selected.

The weight-average molecular mass of the fluid silicone may range from 1,000 to 1, 500,000 g/mol, alternatively 200,000 to 1,000,000 g/mol.

The oil phase of the emulsion can also contain silicone elastomer gels, elastomeric solid organopolysiloxane enclosed in a fatty phase, where at least one elastomeric solid organopolysiloxane is at least partially crosslinked. Examples of such elastomeric solid organopolysiloxane are described in the following Patents and Patent Publications US 5654362, EP 848029, EP 869142, WO2007109240, WO2007109260, WO2007109282, WO2009006091, WO2010080755, US4,987,169, and US5760116. These elastomer gels can be non emulsifying or self emulsifying or a combination of both.

### Hydrophilic Medium

The aqueous phase of the emulsion may comprise a hydrophilic medium comprising water or a mixture of water and a hydrophilic organic solvent, for instance alcohols, such as linear or branched lower monoalcohols containing from 2 to 5 carbon atoms, for instance ethanol, isopropanol or n-propanol, and polyols, for instance glycerol, diglycerol, propylene glycol, sorbitol, pentylene glycol and polyethylene glycols, or alternatively hydrophilic C2 ethers and C2-C4 aldehydes.

The water or the mixture of water and of hydrophilic organic solvents may be present in the emulsion in an amount ranging from 0.1% to 95% by weight and alternatively ranging from 10 % to 80 % by weight relative to the total weight of the emulsion.

### Fillers

The filler suitable for use in the emulsion described herein may be mineral or organic, of any form, platelet-shaped, spherical or oblong, irrespective of the crystallographic form (for example lamellar, cubic, hexagonal, orthorhombic, *etc*.). Examples include talc, mica, silica, kaolin, polyamide, poly-β-alanine powder and polyethylene powder, tetrafluoroethylene polymer powders, lauroyllysine, starch, boron nitride, hollow polymer microspheres, acrylic acid copolymers, silicone resin microbeads, elastomeric polyorganosiloxane particles, precipitated calcium carbonate, magnesium carbonate, magnesium hydrogen carbonate, hydroxyapatite, hollow silica microspheres, glass or ceramic microcapsules, and metal soaps for example zinc stearate, magnesium stearate, lithium stearate, zinc laurate or magnesium myristate, polymethyl methacrylate powders. Alternatively, the filler may be a polyurethane powder.

Alternatively, the filler may be an elastomeric organopolysiloxane powder. Advantageously, the elastomeric organopolysiloxane is non-emulsifying. Spherical elastomeric organopolysiloxanes are described in patent applications JP-A-61-194 009, EP-A-242 219, EP-A-295 886 and EP-A-765 656. The organopolysiloxane powders can also mixed with other particles as described in patent publication U.S. Patent 7,399,803.

The elastomeric organopolysiloxane powder may comprise at least one elastomeric organopolysiloxane powder coated with silicone resin, such as with silsesquioxane resin, as described, for example, in patent US 5,538,793.

Other elastomeric organopolysiloxanes in the form of spherical powders may be hybrid silicone powders functionalized with fluoroalkyl groups or hybrid silicone powders functionalized with phenyl groups.

The filler may be an N-acylamino acid powder. The N- acylamino acids may comprise an acyl group containing from 8 to 22 carbon atoms, for instance a 2-ethylhexanoyl, caproyl, lauroyl, myristoyl, palmitoyl, stearoyl or cocoyl group. The amino acid may be, for example, lysine, glutamic acid or alanine.

When present, the filler may be added to the emulsion in an amount ranging from 0.01 % to 30 % by weight.

### Fibers

For purposes of this application, the term "fiber" means an object of length L and diameter D such that L is very much greater than D , D being the diameter of the circle in which the cross section of the fiber is inscribed.

In particular, the ratio L/D (or shape factor) ranges from 3.5 to 2500, alternatively 5 to 500, and alternatively 5 to 150.

The fiber that may be used in the emulsion may be fibers of synthetic or natural, mineral or organic origin. The fiber that may be used in the emulsion may be selected from polyamide, cellulose, poly-p-phenylene-terephthamide or polyethylene fibers. Polyethylene fibers may also be used.

The fibers may be present in the emulsion in an amount ranging from 0.01 % to 10 % by weight.

### Film-forming polymer

Certain film-forming polymers may be gelling agents. For the purposes of this application, the term "film-forming polymer" means a compound containing at least two repeating units and alternatively at least three repeating units, where said compound is capable, by itself or in the presence of an auxiliary film-forming agent, of forming a macroscopically continuous film on a support, especially on keratin materials, alternatively a cohesive film and alternatively a film with cohesion and mechanical properties such that said film can be isolated from said support.

In one embodiment, the film-forming polymer is a film forming organic polymer chosen from the group comprising: film-forming polymers that are soluble in an organic liquid medium, in particular liposoluble polymers, when the organic liquid medium comprises at least one oil; film-forming polymers that are dispersible in an organic solvent medium, in particular polymers in the form of non-aqueous dispersions of polymer particles, preferably dispersions in silicone oils or hydrocarbon- based oils.

Alternatively, the film-forming polymers that may be used in the emulsion may include synthetic polymers, of free-radical type or of polycondensate type, polymers of natural origin, and mixtures thereof. Such film-forming polymers include acrylic polymers, polyurethanes, polyesters, polyamides, polyureas, cellulose-based polymers, for instance nitrocellulose, silicone polymers, in particular silicone resins, silicone-grafted acrylic polymers, polyamide polymers and copolymers, and polyisoprenes.

The composition according to the invention may comprise, as film-forming polymer, a dispersion of particles of a grafted ethylenic polymer in the fatty phase.

Silicone-based macromonomers that may be used as the film forming polymer include polydimethylsiloxanes containing mono(meth)acrylate end groups. Silicone-based macromonomers that may be used include monomethacryloxypropyl polydimethylsiloxanes.

Alternatively, the emulsion may contain, as film-forming polymer, a linear block ethylenic polymer, referred to hereinbelow as a "block polymer". For purposes of this application, the term "block polymer" means a polymer comprising at least two different blocks and preferably at least three different blocks.

The polymer may be a polymer of linear structure. Alternatively, a polymer of nonlinear structure is, for example, a polymer of branched, star, grafted or other structure may be used.

In one embodiment, the film forming polymer polymer comprises at least three different blocks, and the first and second blocks of the block polymer are mutually incompatible.

In one embodiment, the film-forming polymer is an organic film-forming polymer that is soluble in the fatty phase, which comprises a liquid phase comprising at least one oil.

The liposoluble film forming polymer may be of any chemical type and may especially be chosen from: a ) liposoluble, amorphous homopolymers and copolymers of olefins, of cycloolefins, of butadiene, of isoprene, of styrene, of vinyl ethers, esters or amides, or of (meth) acrylic acid esters or amides comprising a linear, branched or cyclic alkyl group of 4 to 50 carbon atoms, and which may be amorphous. The liposoluble homopolymers and copolymers may be obtained from monomers selected from the group consisting of isooctyl (meth) acrylate, isononyl (meth) acrylate, 2-ethylhexyl (meth) acrylate, lauryl (meth) acrylate, isopentyl (meth) acrylate, n-butyl (meth) acrylate, isobutyl (meth) acrylate, methyl (meth) acrylate, tert-butyl (meth) acrylate, tridecyl (meth) acrylate and stearyl (meth) acrylate, or combinations thereof.

Particular liposoluble copolymers that may be used include: i ) acrylic-silicone grafted polymers containing a silicone backbone and acrylic grafts or containing an acrylic backbone and silicone grafts, such as the product sold under the name SA 70.5 by 3M and described in patents US 5,725,882; US 5,209,924; US 4,972,037; US 4,981,903; US 4,981,902 and US 5,468,477, and in patents US 5,219,560 and EP 0 388 582; ii) liposoluble polymers bearing fluoro groups, belonging to one of the classes described above, in particular the Fomblin products described in patent US 5,948,393 and the alkyl (meth) acrylate/per- fluoroalkyl (meth) acrylate copolymers described in patents EP 0 815 836 and US 5,849,318; iii) polymers or copolymers resulting from the polymerization or copolymerization of an ethylenic monomer, comprising one or more ethylenic bonds, which may be conjugated (or dienes) . A s polymers or copolymers resulting from the polymerization or copolymerization of an ethylenic monomer, it is possible to use vinyl, acrylic or methacrylic copolymers.

In one embodiment, the film-forming polymer is a block copolymer comprising at least one block consisting of styrene units or styrene derivatives (for example methylstyrene, chlorostyrene or chloromethylstyrene).

In one embodiment, the film-forming polymer is selected from copolymers of a vinyl ester (the vinyl group being directly attached to the oxygen atom of the ester group and the vinyl ester having a saturated, linear or branched hydrocarbon-based radical of 1 to 19 carbon atoms, linked to the carbonyl of the ester group) and of at least one other monomer, which may be a vinyl ester (other than the vinyl ester already present), an α-olefin (containing from 8 to 2 8 carbon atoms), an alkyl vinyl ether (the alkyl group of which contains from 2 to 18 carbon atoms) or an allylic or methallylic ester (containing a saturated, linear or branched hydrocarbon-based radical of 1 to 19 carbon atoms, linked to the carbonyl of the ester group).

These copolymers may be partially crosslinked using crosslinking agents, which may be either of the vinyl type or of the allylic or methallylic type, such as tetraallyloxyethane, divinylbenzene, divinyl octanedioate, divinyl dodecanedioate, and divinyl octadecanedioate.

Liposoluble film-forming polymers that may also be mentioned include liposoluble copolymers, such as those resulting from the copolymerization of vinyl esters containing from 9 to 22 carbon atoms or of alkyl acrylates or methacrylates, the alkyl radicals containing from 10 to 2 0 carbon atoms.

Such liposoluble copolymers may be selected from copolymers of polyvinyl stearate, polyvinyl stearate crosslinked with divinylbenzene, with diallyl ether or with diallyl phthalate, polystearyl (meth) acrylate copolymers, polyvinyl laurate and polylauryl (meth) acrylate copolymers, these poly (meth) acrylates possibly being crosslinked with ethylene glycol dimethacrylate or tetraethylene glycol dimethacrylate .

Amorphous and liposoluble polycondensates, preferably not comprising any groups donating hydrogen interactions, in particular aliphatic polyesters containing C4-50 alkyl side chains or polyesters resulting from the condensation of fatty acid dimers, or even polyesters comprising a silicone-based segment in the form of a block, graft or end group, as defined in patent application FR 0 113 920.

Amorphous and liposoluble polysaccharides comprising alkyl (ether or ester) side chains, in particular alkylcelluloses containing a saturated or unsaturated, linear or branched C 1 to C8 alkyl radical, such as ethylcellulose and propylcellulose.

Alternatively, the film-forming polymer may be selected from cellulose-based polymers such as nitrocellulose, cellulose acetate, cellulose acetobutyrate, cellulose acetopropionate or ethylcellulose, or from polyurethanes, acrylic polymers, vinyl polymers, poly vinyl butyrals, alkyd resins, resins derived from aldehyde condensation products, such as arylsulfonamide-formaldehyde resins, for instance toluenesulfonamide-formaldehyde resin, and aryl sulfonamide epoxy resins.

Alternatively, the film forming polymer may be a silicone resin. For purposes of this application, the term "resin" means a three-dimensional structure. In one embodiment, the silicone resin is selected from silsesquioxanes and siloxysilicates. In one embodiment, the silicone resin is selected from siloxysilicates, such as trimethyl siloxysilicates, which are represented by the following formula: [R¹⁶₃SiO_{1/2} ]_{E} (SiO_{4/2})_{F} (units M and Q), in which subscripts E and F may each independently have values ranging from 50 to 80, and R¹⁶ represents an alkyl, such as a methyl or an alkyl of two or more carbon atoms. The ratio of the units M to the units Q may range from 0.7 to 1.

Alternatively, the silicone resin may be selected from silsesquioxanes comprising T units of formula: [R¹⁷SiO_{3/2}]_{G}, in which subscript G has a value that may range up to several thousand and R¹⁷ represents an alkyl, such as a methyl or an alkyl of two or more carbon atoms. In one embodiment, the silsesquioxane is selected from polymethylsilsesquioxanes, which are silsesquioxanes such that R¹⁷ is a methyl group or a propyl group (polypropylsilsesquioxane). The polymethylsilsesquioxanes may comprise, for example, less than 500 T units, and alternatively 50 to 500 T units.

In one embodiment of the invention, the silicone resin is soluble or dispersible in silicone oils or volatile organic liquids. In one embodiment, the silicone resin is solid at 25 °C.

In one embodiment, the silicone resin may have a molecular mass ranging from 1,000 to 10,000 grams/mol.

In another embodiment, the film-forming silicone resin is a copolymer, in which at least one unit of the copolymer is chosen from the silicone units M , D , T and Q , and in which at least one additional unit of the copolymer is chosen from esters.

In a non-limiting manner, the film-forming polymers may be chosen from the following polymers or copolymers: polyurethanes, polyurethane-acrylics, polyureas, polyurea-polyurethanes, polyester-polyure thanes, polyether-polyurethanes, polyesters, polyesteramides, alkyds; acrylic and/or vinyl polymers or copolymers; acrylic-silicone copolymers; polyacrylamides; silicone polymers, for instance silicone polyurethanes or silicone acrylics, and fluoro polymers, and mixtures thereof.

The film forming polymer may be a vinyl polymer comprising at least one carbosiloxane dendrimer-based unit. The vinyl polymer may especially have a backbone and at least one side chain, which comprises a carbosiloxane dendrimer structure. For purposes of this application, the term "carbosiloxane dendrimer structure" represents a molecular structure with branched groups of high molecular masses with high regularity in the radial direction starting from the backbone bond. Such carbosiloxane dendrimer structures are described in the form of a highly branched siloxane-silylalkylene copolymer in the laid-open Japanese patent application Kokai 9-171154.

The vinyl polymer may be one of the polymers described in the examples of patent application EP 0 963 751, or a polymer obtained according to the process described in the said patent application.

According to one embodiment, the vinyl polymer may further comprise at least one organofluorine group. The fluoro vinyl polymer may be one of the polymers described in the examples of patent application WO 03/045 337, or one of polymers obtained according to the process described in said patent application.

According to one embodiment, the grafted vinyl polymers are borne in an oil, which is may be volatile, selected from silicone oils and/or hydrocarbon- based oils. According to one embodiment, the silicone oil may be cyclopentasiloxane. Alternatively, the hydrocarbon-based oil may be isododecane. The emulsion may comprise at least one polyamide polymer or copolymer, which may be selected from polyamide homopolymers, polyamides branched with fatty chains, polyamide-organosiloxanes, polyamide-polyester copolymers and polyamide-polyacrylic copolymers, and mixtures thereof.

As polyamide polymers that may be used in the emulsion, mention may also be made of polyamides comprising at least one polyorganosiloxane group, containing 1 to 1,000 organosiloxane units in the main chain or in the form of a graft. The polyamide polymers are, for example, those described in documents US-A-5 874 069, US-A-5 919 444, US-A-6 051 216, US-A-5 981 680 and WO 04/054 524.

The emulsion may comprise a semi-crystalline polymer, which may have a melting point of greater than or equal to 30 °C. The melting point values correspond to the melting point measured using a differential scanning calorimeter (DSC) such as the calorimeter sold under the name DSC 30 by Mettler, with a temperature rise of 5 or 10 °C per minute. (The melting point considered is the point corresponding to the temperature of the most endothermic peak in the thermogram). The semi-crystalline polymer comprises at least one crystallizable pendent chain or at least one crystallizable block. Aside from the crystallizable chains or blocks, the polymer blocks are amorphous. For the purposes of the invention, the term "crystallizable chain or block" means a chain or block which, if it was alone, would change from the amorphous state to the crystalline state reversibly, depending on whether it is above or below the melting point. For the purposes of this application, a chain is a group of atoms that are pendent or lateral relative to the polymer backbone. A block is a group of atoms belonging to the backbone, this group constituting one of the repeating units of the polymer. The semi-crystalline polymers that may be used in the invention are exemplified by polyolefin block copolymers of controlled crystallization, the monomers of which are described in EP-A-O 951 897.

The film forming polymer, when present, may be in the emulsion in an amount ranging from 0.1 % to 30 % by weight.

### Additional surfactants/ emulsifiers

The emulsion may further comprise an additional surfactant or emulsifier. The additional surfactant or emulsifier may be solid at room temperature, which may be a block polymer, a grafted polymer and/or a random polymer, alone or in combination of two or more. Among the grafted polymers that may be mentioned are silicone polymers grafted with a hydrocarbon-based chain and hydrocarbon- based polymers grafted with a silicone chain.

Thus, grafted-block or block copolymers comprising at least one block of polyorganosiloxane type and at least one block of a free-radical polymer, for instance grafted copolymers of acrylic/silicone type, may be used, which may be used especially when the non-aqueous medium contains silicone.

It is also possible to use grafted-block or block copolymers comprising at least one block of polyorganosiloxane type and at least one block of a polyether. The polyorganopolysiloxane block may be a polydimethylsiloxane or a poly (C2 - C18) alkylmethylsiloxane; the polyether block may be a poly (C2 - C18) alkylene, such as polyoxyethylene and/or polyoxypropylene. In particular, dimethicone copolyols or (C2-C18) alkyldimethicone copolyols may be used.

Water soluble or water dispersible silicone polyether compositions may be included in the present emulsions. These are also known as polyalkylene oxide silicone copolymers, silicone poly (oxyalkylene) copolymers, silicone glycol copolymers, or silicone surfactants. These can be linear, rake, or graft type materials, or ABA type where the B is the siloxane polymer block, and the A is the poly(oxyalkylene) group. The poly(oxyalkylene) group can consist of polyethylene oxide, polypropylene oxide, or mixed polyethylene oxide/polypropylene oxide groups. Other oxides, such as butylene oxide or phenylene oxide are also possible. Another type of silicone polyether composition that may be included in the present composition is an ABn polyalkylene oxide silicone copolymers as described in EP 0 492 657.

The additional emulsifier or surfactant may be selected from nonionic, anionic, cationic and amphoteric surfactants or combinations thereof. Reference may be made to Kirk-Othmer' s "Encyclopedia of Chemical Technology", volume 22, pp. 333-432, 3rd edition, 1979, Wiley, for the definition of the properties and (emulsifying) functions of surfactants, in particular pp. 347-377 of this reference, for anionic, amphoteric and nonionic surfactants.

Nonionic surfactants may be comprise: oxyethylenated and/or oxypropylenated ethers (which may comprise from 1 to 150 oxyethylene and/or oxypropylene groups) of glycerol; oxyethylenated and/or oxypropylenated ethers (which may comprise from 1 to 150 oxyethylene and/or oxypropylene groups) of fatty alcohols (especially of a C8-C24 and alternatively C12-C18 alcohol), such as oxyethylenated cetearyl alcohol ether containing 30 oxyethylene groups (CTFA name Ceteareth-30) and the oxyethylenated ether of the mixture of C12-C15 fatty alcohols comprising 7 oxyethylene groups (CTFA name C12-15 Pareth-7); fatty acid esters (such as a C8-C24 and alternatively C16-C22 acid) of polyethylene glycol (which may comprise from 1 to 150 ethylene glycol units), such as PEG-50 stearate and PEG-40 monostearate; fatty acid esters (especially of a C8-C24 and preferably C16-C22 acid) of oxyethylenated and/or oxypropylenated glyceryl ethers (which may comprise from 1 to 150 oxyethylene and/or oxypropylene groups), for instance PEG-200 glyceryl monostearate; glyceryl stearate polyethoxylated with 30 ethylene oxide groups, glyceryl oleate polyethoxy lated with 30 ethylene oxide groups, glyceryl cocoate polyethoxylated with 30 ethylene oxide groups, glyceryl isostearate polyeth oxylated with 30 ethylene oxide groups, and glyceryl laurate polyethoxylated with 30 ethylene oxide groups; fatty acid esters (especially of a C8-C24 and preferably C16-C22 acid) of oxyethylenated and/or oxypropylenated sorbitol ethers (which may comprise from 1 to 150 oxyethylene and/or oxypropylene groups),; dimethicone copolyol benzoate; copolymers of propylene oxide and of ethylene oxide, also known as EO/PO polycondensates; and mixtures thereof; saccharide esters and ethers, such as sucrose stearate, sucrose cocoate and sorbitan stearate, and mixtures thereof, fatty acid esters (such as a C8-C24 and alternatively C16-C22 acid) of polyols, especially of glycerol or of sorbitol, such as glyceryl stearate, glyceryl stearate, glyceryl laurate, polyglyceryl-2 stearate, sorbitan tristearate or glyceryl ricinoleate.

Anionic surfactants include C16-C30 fatty acid salts, such as those derived from amines, for instance triethanolamine stearate; polyoxyethylenated fatty acid salts, such asthose derived from amines or alkali metal salts, and combinations thereof; phosphoric esters and salts thereof, such as DEA oleth-10 phosphate or monocetyl monopotassium phosphate sulf osuccinates such as Disodium PEG-5 citrate lauryl sulf osuccinate and Disodium ricinoleamido MEA sulf osuccinate; alkyl ether sulfates, such as sodium lauryl ether sulfate; isethionates; acylglutamates such as disodium hydrogenated tallow glutamate, alkyl polyglucosides and combinations thereof.

The emulsion may further comprise an amphoteric surfactant, for instance N-acylamino acids such as N-alkylaminoacetates and disodium cocoamphodiacetate, and amine oxides such as stearamine oxide, or alternatively silicone surfactants, for instance dimethicone copolyol phosphates.

### Dyestuffs

The emulsion may further comprise a dyestuff. The dyestuff may be selected from pulverulent dyestuffs (such as pigments and nacres) and water-soluble dyestuffs. For purposes of this application, the term "pigments" means white or colored, mineral or organic particles of any form, which are insoluble in the physiological medium, and which are intended to color the emulsion. For purposes of this application, the term "nacres" means iridescent particles of any form, produced especially by certain molluscs in their shell, or else synthesized.

The pigments may be white or colored, and mineral and/or organic. The mineral pigments include titanium dioxide, optionally surface-treated, zirconium oxide or cerium oxide, zinc oxide, iron oxide (black, yellow or red), chromium oxide, manganese violet, ultramarine blue, chromium hydrate, ferric blue, and metal powders, for instance aluminum powder or copper powder. The organic pigments include carbon black, pigments of D & C type, and lakes based on cochineal carmine or on barium, strontium, calcium or aluminum.

Mention may also be made of pigments with an effect, such as particles comprising a natural or synthetic, organic or mineral substrate, for example glass, acrylic resins, polyester, polyurethane, polyethylene terephthalate, ceramics or aluminas, said substrate being uncoated or coated with metallic substances, for instance aluminum, gold, silver, platinum, copper or bronze, or with metal oxides, for instance titanium dioxide, iron oxide or chromium oxide, and combinations thereof.

The nacres may be chosen from white nacreous pigments such as mica coated with titanium or with bismuth oxychloride, colored nacreous pigments such as titanium mica coated with iron oxides, titanium mica coated with ferric blue or with chromium oxide, titanium mica coated with an organic pigment of the abovementioned type, and also nacreous pigments based on bismuth oxychloride. Interference pigments, such as liquid-crystal or multilayer interference pigments, may alternatively be used.

### Structuring agents

The emulsion may further comprise a structuring agent. For purposes of this application, the term "structuring agent" means a compound capable of increasing the viscosity of the emulsion. The structuring agent makes it possible to obtain an emulsion that can have a texture ranging from fluid to solid textures.

The structuring agent may be present in the emulsion in an amount ranging from 0.1 % to 20 % by weight, alternatively ranging from 0.1 % to 15 % by weight and alternatively ranging from 0.5 % to 10 % by weight, relative to the total weight of the emulsion.

The structuring agent may be selected from thickeners (oily-medium thickeners; aqueous-medium thickeners), organogelling agents, waxes, pasty compounds and gums.

The aqueous-medium thickener may be chosen from: hydrophilic clays, hydrophilic fumed silica, water-soluble cellulose-based thickeners, guar gum, xanthan gum, carob gum, scleroglucan gum, gellan gum, rhamsan gum, karaya gum or carrageenan gum, alginates, maltodextrins, starch and its derivatives, and hyaluronic acid and its salts, the polyglyceryl (meth) acrylate polymers sold under the names Hispagel or Lubragel by Hispano Quimica or Guardian, polyvinylpyrrolidone, polyvinyl alcohol, crosslinked acrylamide polymers and copolymers, or alternatively the crosslinked methacryloyloxyethyltrimethylammonium chloride homopolymers, associative polymers and especially associative polyurethanes and sodium acrylate blends. Such thickeners are described especially in patent application EP-A-1 400 234.

The oily-medium thickener may be chosen from: organophilic clays; hydrophobic fumed silicas; alkyl guar gums (with a C1-C 6 alkyl group), such as those described in EP-A-708 114; oil-gelling polymers, for instance triblock polymers or star polymers resulting from the polymerization or copolymerization of at least one monomer containing an ethylenic group; .

Alternatively, the structuring agent can be a wax. For the purposes of this application, the term "wax" means a lipophilic compound that is solid at room temperature (25 °C), which undergoes a reversible solid/liquid change of state, and which has a melting point of greater than or equal to 30 °C , which may be up to 120 °C .

The waxes may be hydrocarbon-based waxes, fluoro waxes and/or silicone waxes, and may be of plant, mineral, animal and/or synthetic origin. In particular, the waxes may have a melting point of greater than 30 °C .

Suitable waxes include beeswax, carnauba wax or candelilla wax, paraffin, microcrystalline waxes, ceresin or ozokerite; synthetic waxes, for instance polyethylene waxes or Fischer-Tropsch waxes, and silicone waxes, for instance alkyl, alkoxy dimethicones containing from 16 to 45 carbon atoms or silsesquioxane resin wax as described in patent application publication WO2005100444.

Alternatively, the emulsion may contain a pasty compound, which may be selected from lanolin and its derivatives; polymeric or non-polymeric silicone compounds; polymeric or non-polymeric fluoro compounds; vinyl polymers, such as olefin homopolymers, olefin copolymers, hydrogenated diene homopolymers, and linear or branched oligomers, homopolymers or copolymers of alkyl (meth) acrylates, such as those containing a C8-C30 alkyl group; oligomers, homopolymers, and copolymers of vinyl esters containing C8-C30 alkyl groups; oligomers, homopolymers and copolymers of vinyl ethers containing C8-C30 alkyl groups; liposoluble polyethers resulting from the polyetherification between one or more C2-C100 (alternatively C2-C50) diols, esters, and combinations thereof. The esters include esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid. The pasty compounds of plant origin include a mixture of soybean sterols and of oxyethylenated (5 OE) oxypropylenated (5 OP) pentaerythritol.

### Active Ingredients

As used herein, a "personal care active" means any compound or combination of compounds that are known in the art as additives in the personal care formulations that are typically added for the purpose of treating hair or skin to provide a cosmetic and/or aesthetic benefit. A "healthcare active" means any compound or mixtures of compounds that are known in the art to provide a pharmaceutical or medical benefit. Thus, "healthcare active" include materials consider as an active ingredient or active drug ingredient as generally used and defined by the United States Department of Health & Human Services Food and Drug Administration, contained in Title 21, Chapter I, of the Code of Federal Regulations, Parts 200-299 and Parts 300-499.

Some representative examples of active ingredients include; drugs, vitamins, minerals; hormones; topical antimicrobial agents such as antibiotic active ingredients, antifungal active ingredients for the treatment of athlete's foot, jock itch, or ringworm, and acne active ingredients; astringent active ingredients; deodorant active ingredients; wart remover active ingredients; corn and callus remover active ingredients; pediculicide active ingredients for the treatment of head, pubic (crab), and body lice; active ingredients for the control of dandruff, seborrheic dermatitis, or psoriasis; and sunburn prevention and treatment agents.

Useful active ingredients for use in the emulsion include vitamins and their derivatives, including "pro-vitamins". Vitamins useful herein include, but are not limited to, Vitamin A₁, retinol, C2 to C18 esters of retinol, vitamin E, tocopherol, esters of vitamin E, and combinations thereof. Retinol includes trans-retinol, 1, 3-cis-retinol, 11-cis-retinol, 9-cis-retinol, and 3,4-didehydro-retinol, Vitamin C and its derivatives, Vitamin B₁, Vitamin B₂, Pro Vitamin B5, panthenol, Vitamin B₆, Vitamin B₁₂, niacin, folic acid, biotin, and pantothenic acid. Other suitable vitamins and the International Nomenclature Cosmetic Ingredient Name (INCI) names for the vitamins considered included herein are ascorbyl dipalmitate, ascorbyl methylsilanol pectinate, ascorbyl palmitate, ascorbyl stearate, ascorbyl glucocide, sodium ascorbyl phosphate, sodium ascorbate, disodium ascorbyl sulfate, potassium (ascorbyl/tocopheryl) phosphate.

Alternatively, the active ingredient used in the emulsion can be an active drug ingredient. Representative examples of some suitable active drug ingredients which can be used are hydrocortisone, ketoprofen, timolol, pilocarpine, adriamycin, mitomycin C, morphine, hydromorphone, diltiazem, theophylline, doxorubicin, daunorubicin, heparin, penicillin G, carbenicillin, cephalothin, cefoxitin, cefotaxime, 5-fluorouracil, cytarabine, 6-azauridine, 6-thioguanine, vinblastine, vincristine, bleomycin sulfate, aurothioglucose, suramin, mebendazole, clonidine, scopolamine, propranolol, phenylpropanolamine hydrochloride, ouabain, atropine, haloperidol, isosorbide, nitroglycerin, ibuprofen, ubiquinones, indomethacin, prostaglandins, naproxen, salbutamol, guanabenz, labetalol, pheniramine, metrifonate, and steroids.

Considered to be included herein as active drug ingredients for purposes of this application are antiacne agents such as benzoyl peroxide and tretinoin; antibacterial agents such as chlorohexadiene gluconate; antifungal agents such as miconazole nitrate; anti-inflammatory agents; corticosteroidal drugs; non-steroidal anti-inflammatory agents such as diclofenac; antipsoriasis agents such as clobetasol propionate; anesthetic agents such as lidocaine; antipruritic agents; antidermatitis agents; and agents generally considered barrier films.

Alternatively, the active ingredient in the emulsion can be a protein, such as an enzyme. Enzymes include, but are not limited to, commercially available types, improved types, recombinant types, wild types, variants not found in nature, and mixtures thereof. For example, suitable enzymes include hydrolases, cutinases, oxidases, transferases, reductases, hemicellulases, esterases, isomerases, pectinases, lactases, peroxidases, laccases, catalases, and mixtures thereof. Hydrolases include, but are not limited to, proteases (bacterial, fungal, acid, neutral or alkaline), amylases (alpha or beta), lipases, mannanases, cellulases, collagenases, lisozymes, superoxide dismutase, catalase, and mixtures thereof. Said proteases include, but are not limited to, trypsin, chymotrypsin, pepsin, pancreatin and other mammalian enzymes; papain, bromelain and other botanical enzymes; subtilisin, epidermin, nisin, naringinase(L-rhammnosidase) urokinase and other bacterial enzymes. Said lipases include, but are not limited to, triacyl-glycerol lipases, monoacyl-glycerol lipases, lipoprotein lipases, e.g., steapsin, erepsin, pepsin, other mammalian, botanical, bacterial lipases and purified ones. Natural papain is useful as said enzyme. Further, stimulating hormones, e.g., insulin, can be used together with these enzymes to boost the effectiveness of them.

Alternatively, the active ingredient may be a sunscreen agent. The sunscreen agent can be selected from any sunscreen agent known in the art to protect skin from the harmful effects of exposure to sunlight. The sunscreen agent may be selected from an organic compound, an inorganic compound, or a combination thereof that absorbs ultraviolet (UV) light. Representative, non-limiting examples that can be used as the sunscreen agent include; Aminobenzoic Acid, Cinoxate, Diethanolamine Methoxycinnamate, Digalloyl Trioleate, Dioxybenzone, Ethyl 4-[bis(Hydroxypropyl)] Aminobenzoate, Glyceryl Aminobenzoate, Homosalate, Lawsone with Dihydroxyacetone, Menthyl Anthranilate, Octocrylene, Octyl Methoxycinnamate, Octyl Salicylate, Oxybenzone, Padimate O, Phenylbenzimidazole Sulfonic Acid, Red Petrolatum, Sulisobenzone, Titanium Dioxide, and Trolamine Salicylate, cetaminosalol, Allatoin PABA, Benzalphthalide, Benzophenone, Benzophenone 1-12, 3-Benzylidene Camphor, Benzylidenecamphor Hydrolyzed Collagen Sulfonamide, Benzylidene Camphor Sulfonic Acid, Benzyl Salicylate, Bornelone, Bumetriozole, Butyl Methoxydibenzoylmethane, Butyl PABA, Ceria/Silica, Ceria/Silica Talc, Cinoxate, DEA-Methoxycinnamate, Dibenzoxazol Naphthalene, Di-t-Butyl Hydroxybenzylidene Camphor, Digalloyl Trioleate, Diisopropyl Methyl Cinnamate, Dimethyl PABA Ethyl Cetearyldimonium Tosylate, Dioctyl Butamido Triazone, Diphenyl Carbomethoxy Acetoxy Naphthopyran, Disodium Bisethylphenyl Tiamminotriazine Stilbenedisulfonate, Disodium Distyrylbiphenyl Triaminotriazine Stilbenedisulfonate, Disodium Distyrylbiphenyl Disulfonate, Drometrizole, Drometrizole Trisiloxane, Ethyl Dihydroxypropyl PABA, Ethyl Diisopropylcinnamate, Ethyl Methoxycinnamate, Ethyl PABA, Ethyl Urocanate, Etrocrylene Ferulic Acid, Glyceryl Octanoate Dimethoxycinnamate, Glyceryl PABA, Glycol Salicylate, Homosalate, Isoamyl p-Methoxycinnamate, Isopropylbenzyl Salicylate, Isopropyl Dibenzolylmethane, Isopropyl Methoxycinnamate, Menthyl Anthranilate, Menthyl Salicylate, 4-Methylbenzylidene, Camphor, Octocrylene, Octrizole, Octyl Dimethyl PABA, Octyl Methoxycinnamate, Octyl Salicylate, Octyl Triazone, PABA, PEG-25 PABA, Pentyl Dimethyl PABA, Phenylbenzimidazole Sulfonic Acid, Polyacrylamidomethyl Benzylidene Camphor, Potassium Methoxycinnamate, Potassium Phenylbenzimidazole Sulfonate, Red Petrolatum, Sodium Phenylbenzimidazole Sulfonate, Sodium Urocanate, TEA-Phenylbenzimidazole Sulfonate, TEA-Salicylate, Terephthalylidene Dicamphor Sulfonic Acid, Titanium Dioxide, Zinc Dioxide, Serium Dioxide, TriPABA Panthenol, Urocanic Acid, and VA/Crotonates/Methacryloxybenzophenone-1 Copolymer. These sunscreen agents can be selected as one or a combination of two or more.

Alternatively, the active ingredient may a plant extract. Altermatively, the active ingredient may be a self tanning agent such as but not limited to dihydroxyacetone and erythrulose or an insect repellent such as but not limited to ethyl butylacetylaminopropionate or plant extract such as citronella. The amount of active ingredient present in the emulsion will vary depending on factors including the type of active ingredient selected and the method of use of the emulsion, however, the amount of active ingredient may range from 0.05 wt % to 50 wt %, alternatively 1 wt % to 25 wt %, or alternatively 1 to 10 wt %, based on the weight of the emulsion.

Alternatively, the active ingredient may be an antiperspirant and/or deodorant agent. Some examples of antiperspirant agents and deodorant agents are Aluminum Chloride, Aluminum Zirconium Tetrachlorohydrex GLY, Aluminum Zirconium Tetrachiorohydrex PEG, Aluminum Chlorohydrex, Aluminum Zirconium Tetrachiorohydrex PG, Aluminum Chlorohydrex PEG, Aluminum Zirconium Trichlorohydrate, Aluminum Chiorohydrex PG, Aluminum Zirconium Trichlorohydrex GLY, Hexachlorophene, Benzalkonium Chloride, , Aluminum Sesquichlorohydrate, Sodium Bicarbonate, Aluminum Sesquichlorohydrex PEG, ,Chlorophyllin-Copper Complex, Triclosan, Aluminum Zirconium Octachlorohydrate, and Zinc Ricinoleate.

### Fragrance

Fragrance or perfume can also be added to the emulsion. The fragrance can be any perfume or fragrance ingredient commonly used in the perfume industry. These fragrance ingredients may belong to a variety of chemical classes, as varied as alcohols, aldehydes, ketones, esters, ethers, acetates, nitrites, terpenic hydrocarbons, heterocyclic nitrogen or sulfur containing compounds, as well as essential oils of natural or synthetic origin. Many of these fragrance ingredients are described in detail in standard textbook references such as Perfume and Flavour Chemicals, 1969, S. Arctander, Montclair, N.J.

### Preservatives

When making an emulsion with the emulsifiers described herein, it may be desirable to add various preservatives such as the parabens, BHT, BHA, phenoxy ethanol, as listed on the Annex VI, Part 1 of the European Cosmetic directive - LIST OF PRESERVATIVES WHICH COSMETIC PRODUCTS MAY CONTAIN. When present, the amount of preservative may range from 0.01 % to 5 % by weight based on the weight of the emulsion.

The emulsion is suitable for use in personal care products. Such personal care products are exemplified by antiperspirants and deodorants, skin creams, skin care lotions, moisturizers, facial treatments such as acne or wrinkle removers, personal and facial cleansers, bath oils, perfumes, colognes, sachets, sunscreens, pre-shave and after-shave lotions, shaving soaps, and shaving lathers, hair shampoos, hair conditioners, hair colorants, hair relaxants, hair sprays, mousses, gels, permanents, depilatories, and cuticle coats, make-ups, color cosmetics, foundations, concealers, blushes, lipsticks, eyeliners, mascara, oil removers, color cosmetic removers, wrinkle fillers, skin imperfection hiders, skin surface smoothers, eyelash curlers, nail varnishes, hair make-up products, eye shadows, body makeups, and powders, medicament creams, pastes or sprays including anti-acne, dental hygienic, antibiotic, healing promotive, nutritive and the like, which may be preventative and/or therapeutic.

### EXAMPLES

The following examples are included to demonstrate the invention to one of ordinary skill. However, those of ordinary skill in the art should, in light of the present disclosure, appreciate that many changes can be made in the specific embodiments which are disclosed and still obtain a like or similar result without departing from the invention as defined by the amended claims. All amounts, ratios, and percentages are by weight unless otherwise indicated.

### Reference Example A - Preparation of Comparative Copolymers 1-3

Copolymer samples were prepared by reacting a trimethylsiloxy terminated poly(dimethyl/ methyl(aminoethylaminoisobutyl)siloxane), with a viscosity of 3,000 cP and an average of 4 aminoethylaminoisobutyl groups per molecule (20 g) with delta gluconolactone (1.1 g) in the presence of ethanol (21 g) by heating to 74 °C for 4 hours while stirring continuously under reflux. The solvent was then removed by vacuum stripping. After the solvent was removed the resulting copolymer was in the form of a gum.

### Comparative Example 1:

An oil, 5 cSt polydimethylsiloxane (DOW CORNING® 200 Fluid) in an amount of 180 g and 20 g of the gum described in Reference Example A, were combined and stirred at room temperature. The gum failed to dissolve in the polydimethylsiloxane after two days, and no emulsion was formed.

### Comparative Example 2

An oil, 5 cSt polydimethylsiloxane (DOW CORNING® 200 Fluid) in an amount of 180 g; 20 g of the gum described in Reference Example A; and 20 g ethanol were combined and stirred at room temperature. It took several days for the resulting mixture to become a clear solution. This solution was the oil phase.

After the clear solution formed, 80 g of aqueous phase was added. The aqueous phase was prepared by mixing 74 weight parts deionized water, 5 parts glycerol, and 1 part sodium chloride until a clear solution formed. The aqueous phase was added incrementally (in 5 g increments) to 20 g of the oil phase with mixing in between additions using a FlackTek mixer. After an emulsion formed, ethanol was removed by stripping at 50 °C under full vacuum. Overall, in this example, several days were required to prepare an emulsion.

### Comparative Example 3

An oil, 5 cSt polydimethylsiloxane (DOW CORNING@ 200 Fluid) in an amount of 180 g and 20 g of the gum described in Reference Example A, were combined and stirred at 70 °C. It took several days for the mixture to form a clear solution. After the clear solution formed, 80 g aqueous phase was added as in comparative example 2. Overall, in this example, several days were required to prepare an emulsion.

### Example 4

Copolymer samples were prepared by reacting a trimethylsiloxy terminated poly(dimethyl/ methyl(aminoethylaminoisobutyl)siloxane), with a viscosity of 3,000 cP and an average of 4 aminoethylaminoisobutyl groups per molecule (20 g) with delta gluconolactone (1.1 g) in the presence of ethanol (21 g) by heating to 74 °C for 4 hours while stirring continuously under reflux. The resulting product was mixed with 5 cSt polydimethylsiloxane (DOW CORNING@ 200 Fluid) in an amount of 180 g. Ethanol was then removed by vacuum stripping at 74 °C under full vacuum. The resulting copolymer/oil combination was a liquid.

After the ethanol was removed, 80 g aqueous phase was added to the copolymer/oil combination. The aqueous phase and method of addition was as in comparative example 2. An emulsion formed within minutes. Example 4 shows that the total time to prepare an emulsion is much faster when the method described herein is used.

### Examples 5 and 6

A copolymer of formula was used in the following examples: 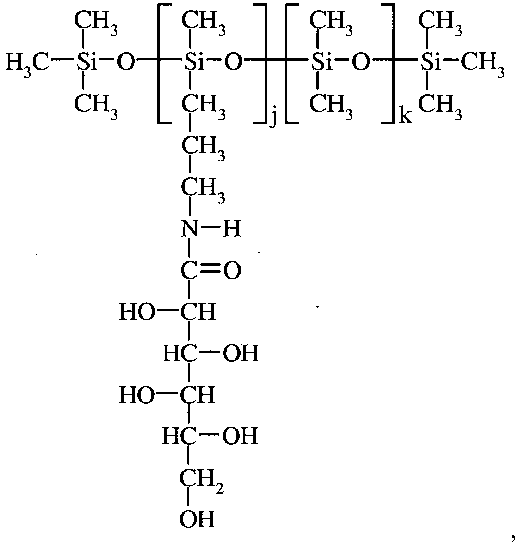 where subscript j had a value ranging from 3 to 5 and subscript k had a value ranging from 250 to 450.

In example 5, the copolymer was tested for compatibility with various oils by visually observing mixtures prepared by mixing 1 weight part copolymer with 9 weight parts oil. For comparison, Abil 90 EM (which is commercially available from Evonik) was also mixed with the oils in a 1/9 weight ratio. The results are as follows.

| | Copolymer | Abil 90 EM |
|---|---|---|
| 5 cSt 200 Fluid | clear | hazy |
| FZ-3196 | clear | clear |
| Isohexadecane | clear | clear |
| Isopropyl Myristate | immiscible | clear |
| Alkyl Benzoate | immiscible | clear |
| Capric Triglyceride | immiscible | clear |
| Mineral Oil | immiscible | clear |

In example 6, the copolymer was used as an emulsifier to make emulsions. In the tables below, Crodamol GTCC refers to a medium chain triglyceride of low viscosity, which is used as an emollient. Crodamol GTCC is commercially available from Croda, Inc. of Edison, New Jersey, U.S.A. The emulsions in Table 1 were prepared using a dental mixer. The emulsions in Table 2 were prepared using a stirrer mixer. The procedures for preparing these emulsions were as follows.

### Reference Example B - Process for making an emulsion with stirrer mixer

Emulsions containing the ingredients in Table 3 using the copolymer as emulsifier were prepared by the following method:
1. The ingredients of phase A were mixed together to obtain a homogeneous mix.
2. The ingredients of phase B were mixed together to obtain a homogeneous mix
3. Phase B was added to phase A under mixing (with a cross stirrer). While adding phase B, mixing speed increased from 700 rpm to 1000 rpm.
4. After all phase B was added, the resulting product was mixed for 1 minute at 1000 rpm and 5 minutes at 2000 rpm. A coarse emulsion was obtained.
5. A 100 gram sample of the coarse emulsion was passed through a high shear mixing apparatus for 15 seconds to reduce the particle size. The high shear mixing apparatus was a lab mixer from Silverson Machines Ltd. of England. A fine emulsion was obtained.

### Reference Example C - Process for making an emulsion with dental mixer

Emulsions containing the ingredients in Table 1 using a copolymer as emulsifier were prepared by the following method:
1. The ingredients of phase A were mixed together.
2. The ingredients of phase B were mixed together.
3. Phase B was added to phase A in 5 g increments.
4. After addition of each increment, the resulting product was mixed for 40 seconds at 3400 rpm in a dental mixer (DAC 150 Series - SpeedMixer^{™}).
5. Steps 3 and 4 were repeated until all phase B was added to obtain a final emulsion.

### Reference Example D - Emulsion Stability

Stability of the emulsions prepared herein was evaluated during storage of samples of each emulsion for 6 months at room temperature (RT), 40°C, and 50°C. Stability was measured by visual inspection. The results are in the tables below.

### Reference Example E - Freeze/Thaw stability of emulsions

Samples of the emulsions prepared herein were evaluated for freeze/thaw stability. The procedure was as follows:
1. Emulsion samples were refrigerated at 4 °C for a minimum of 12 hours and then stored at RT for few hours.
2. Emulsion stability was evaluated. Stability was measured by visual inspection.
3. Steps 1 and 2 were repeated five times. The results are in the tables below.

**Table 1**

| | Sample Number | 6-1 | 6-2 | 6-3 | 6-4 | 6-5 | 6-6 |
|---|---|---|---|---|---|---|---|
| | Ingredient / Amount in wt % for each sample | % | % | % | % | % | % |
| **Phase A** | Mixture of 85 % 5 cSt 200 Fluid and 15 % Copolymer | 6.67 | 13.33 | 13.33 | 13.33 | 13.33 | 6.67 |
| | Xiameter® PMX-200 Silicone Fluid 5 cSt | 13.33 | 6.67 | / | / | / | / |
| | Xiameter® PMX-200 Silicone Fluid 2 cSt | / | / | 6.67 | / | / | / |
| | Xiameter® PMX-Silicone 200 Fluid 5 cSt/ Crodamol GTCC (50 %/50 % mixture by weight) | / | / | / | 6.67 | 6.667 | 33.33 |
| | Mineral Oil | / | // | / | / | / | / |
| **Phase B** | Water | 74 | 74 | 74 | 74 | 74 | 54 |
| | Glycerin | 5 | 5 | 5 | 5 | 5 | 5 |
| | NaCl | 1 | 1 | 1 | 1 | 1 | 1 |

**Table 2**

| Viscosity (Brookfield DV-II-Spindle 6, 2,5 rpm) | | | | | | |
|---|---|---|---|---|---|---|
| 1 day | / | / | / | / | 286000 | 34400 |
| 1 week | / | / | / | / | 271000 | / |
| 2 weeks | / | / | / | / | 268000 | / |
| 3 weeks | / | / | / | / | 261000 | / |
| 1 month | / | / | / | / | 247000 | / |
| 2 months | / | / | / | / | 245000 | / |
| 3 months | / | / | / | / | 245000 | / |
| 4 months | / | / | / | / | / | / |

| Stability | | | | | | |
|---|---|---|---|---|---|---|
| At RT | At least 3 months | At least 3 months | Stable for 1 month | At least 3 months | At least 3 months | Stable for 1 day |
| At 40 °C | At least 3 months | At least 3 months | At least 3 months | At least 3 months | At least 3 months | Stable for 2 weeks |
| At 50 °C | / | / | / | / | At least 3 months | Stable for 2 weeks |
| F/T Cycle | / | / | / | / | passed 1 cycle | 0 |
| Sample Number | 6-1 | 6-2 | 6-3 | 6-4 | 6-5 | 6-6 |

**Table 3**

| | Sample Number | 6-7 | 6-8 | 6-9 | 6-10 | 6-11 | 6-12 | 6-13 |
|---|---|---|---|---|---|---|---|---|
| | Ingredient / Amount in wt % for each sample | % | % | % | % | % | % | % |
| **Phase A** | Mixture of 85 % 5 cSt 200 Fluid and 15 % Copolymer | 13.33 | 6.67 | 13.33 | 13.33 | 6.67 | 13.33 | 6.67 |
| | Xiameter® PMX-200 Silicone Fluid 5 cSt | / | / | / | / | 33.33 | 26.33 | / |
| | Xiameter® PMX-Silicone 200 Fluid 5cSt/ Crodamol GTCC (50 %/50 % mixture by weight) | 6.6 67 | 33.33 | 26.33 | 33.33 | / | / | 3.33 |
| **Phase B** | Water | 74 | 54 | 54 | 54 | 54 | 54 | 84 |
| | Glycerin | 5 | 5 | 5 | 5 | 5 | 5 | 5 |
| | NaCl | 1 | 1 | 1 | 1 | 1 | 1 | 1 |

**Table 4**

| Viscosity (Brookfield DV-II-Spindle 6, 2,5 rpm) | | | | | | | |
|---|---|---|---|---|---|---|---|
| 1 day | 286000 | 34400 | 26600 | 31200 | 1200 | 1200 | Broken during process |
| 1 week | 271000 | / | / | / | / | / | / |
| 2 weeks | 268000 | / | / | / | / | / | / |
| 3 weeks | 261000 | / | / | / | / | / | / |
| 1 month | 247000 | / | / | / | / | / | / |
| 2 months | 245000 | / | / | / | / | / | / |
| 3 months | 245000 | / | / | / | / | / | / |
| 4 months | | / | / | / | / | / | / |

| Stability | | | | | | | |
|---|---|---|---|---|---|---|---|
| At RT | At least 3 months | Stable for 1 week | Stable for 1 week | Stable for 1 week | Stable for 1 week | Stable for 1 week | / |
| At 40 °C | At least 3 months | Stable for 2 weeks | / | Stable for 1 week | Stable for 1 week | Stable for 1 week | / |
| At 50 °C | At least 3 months | Stable for 2 weeks | / | Stable for 1 week | Stable for 1 week | Stable for 1 week | / |
| F/T Cycle | 0 | 0 | 0 | 0 | 0 | 0 | / |
| Sample Number | 6-7 | 6-8 | 6-9 | 6-10 | 6-11 | 6-12 | 6-13 |

### Industrial Applicability

The inventors surprisingly found that an emulsion can be prepared much more quickly using the method described herein than using previously known processes. Without wishing to be bound by theory, it is thought that the emulsion may also have improved performance when made by the method herein.

## Claims

1. A method comprising:
i) preparing a saccharide siloxane copolymer in the presence of a solvent, where the saccharide siloxane copolymer has formula
R²ₐR¹₍₃₋ₐ₎SiO-[(R²R¹SiO)ⱼ(R¹₂SiO)ₖ]_{y}-SiR¹₍₃₋ₐ₎R²ₐ;
where
each subscript a is independently 0, 1, 2, or 3;
each subscript j is independently an integer with a value ranging from 0 to 10,000;
each subscript k is independently an integer with a value ranging from 0 to 10,000;
subscript y is an integer such that the copolymer has molecular weight up to 1,000,000;
each R¹ can be the same or different and comprises hydrogen, an alkyl group, an organic radical, or a group of formula R³-Q;
Q comprises an epoxy, cycloalkyepoxy, primary or secondary amino, ethylenediamine, carboxy, halogen, vinyl, allyl, anhydride, or mercapto functionality;
each R² has the formula Z-(G¹)ₙ-(G²)ₒ, and there is an average of at least one R² per polymer molecule;
G¹ is a saccharide component comprising 5 to 12 carbon atoms;
a quantity (n + o) is 1 to 10, and
subscript n or subscript o can be 0;
G² is a saccharide component comprising 5 to 12 carbon atoms, and G² is additionally substituted with an organic radical or an organosilicon radical;
each Z is a linking group independently selected from the group consisting of
-R³-NHC(O)-R⁴-;
-R³-NHC(O)O-R⁴-;
-R³-NH-C(O)-NH-R⁴-;
-R³-C(O)-O-R⁴-;
-R³-O-R⁴-;
-R³-CH(OH)-CH₂-O-R⁴ -;
-R³-S-R⁴-;
-R³-CH(OH)-CH₂-NH-R⁴-;
-R³-N(R¹)-R⁴-;
-NHC(O)-R⁴-;
-NHC(O)O-R⁴-;
-NH-C(O)-NH-R⁴-;
-C(O)-O-R⁴-;
-O-R⁴-;
-CH(OH)-CH₂-O-R⁴-;
-S-R⁴-;
-CH(OH)-CH₂-NH-R⁴-;
-N(R¹)-R⁴-;
-R³-NHC(O)-;
-R³-NHC(O)O-;
-R³-NH-C(O)-NH-;
-R³-C(O)-O-;
-R³-O-;
-R³-CH(OH)-CH₂-O-;
-R³-S-;
-R³-CH(OH)-CH₂-NH-; and
-R³-N(R¹)-;
where each R³ and each R⁴ is independently a divalent spacer comprising a group of formula (R⁵)ᵣ(R⁶)ₛ(R⁷)ₜ,
where at least one of subscripts r, s and t is 1; each R⁵ and each R⁷ are independently either an alkyl group of 1 to 12 carbon atoms or a group of formula (R⁹O)ₚ, where R⁹ is a hydrocarbon group of 1 to 12 carbon atoms, subscript p is an integer ranging from 1 to 50, and each R⁹O may be the same or different;
R⁶ is -N(R⁸)-, where R⁸ is hydrogen, an alkyl group of 1 to 12 carbon atoms, a group of formula Z-X, or R³; and
each X is independently a carboxylic acid, phosphate, sulfate, sulfonate or quaternary ammonium radical;
optionally ii) removing a portion of the solvent; and
iii) adding an oil,
wherein the oil is added before and/or during step i).

2. The method of claim 1,
where each subscript j is independently an integer with a value ranging from 0 to 500 and each subscript k is independently an integer with a value ranging from 0 to 500 or
where each R⁵ and each R⁷ are independently an alkyl group of 1 to 12 carbon atoms or
further comprising iv) removing the solvent or
where step i) is performed by reacting ingredients comprising:
(A) an amino-functional polyorganosiloxane, and
(B) a sugar lactone or
where the product of step i) contains secondary amine functionality, further comprising a step of reacting the product of step i) with a capping agent selected from a lactone, a halogenated unsaturated compound, an epoxy functional compound, or an acid anhydride or
where the solvent is an alcohol selected from methanol, ethanol, n-propanol, isopropanol, 2-propanol, isobutanol, n-butanol, and combinations thereof or where the oil is an organic oil selected from a hydrocarbon oil, an ester, a vegetable oil, a mineral oil, or a fatty alcohol or
where the copolymer and the oil are present in an amounts such that a weight ratio of copolymer/oil ranges from 1/1 to 1/50.

3. The method of claim 1, where ingredient (A) is 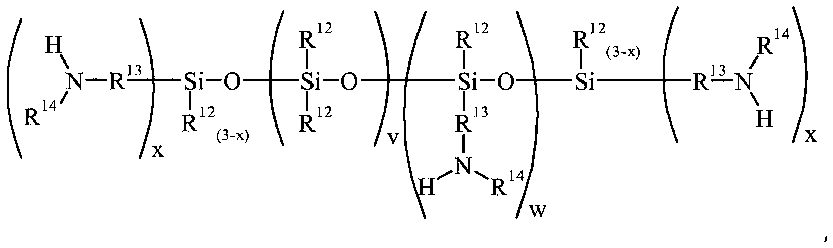 where each R¹² is independently a monovalent hydrocarbon group;
each R¹³ is independently a divalent organic group;
each R¹⁴ is independently a hydrogen atom or a monovalent hydrocarbon group of 1 to 4 carbon atoms;
each subscript x is independently 0 or 1;
subscript v has a value ranging from 0 to 10,000; and
subscript w has a value ranging from 0 to 10,000 and preferably
where ingredient (A) is selected from trimethylsiloxy-terminated poly(dimethylsiloxane/methyl(aminoethylaminoisobutyl)siloxane), trimethylsiloxy-terminated poly(dimethylsiloxane/methyl(aminopropyl)siloxane), trimethylsiloxy-terminated poly(dimethylsiloxane/methyl(aminoethylaminopropyl)siloxane), and combinations thereof.

4. The method of claim 1, where ingredient (B) is 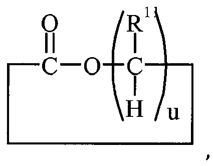 where each R¹¹ is independently a hydrogen atom, a hydroxyl group, an alkoxy group, or a saccharide group; and
subscript u has a value ranging from 5 to 12 and preferably
where ingredient (B) is selected from butyrolactone, epsilon caprolactone and delta gluconolactone.

5. The method of claim 1, where step i) is performed by reacting ingredients comprising:
(A) an epoxy-functional polyorganosiloxane, and
(B) an n-alkyl glucamine.

6. The method of claim 5, where ingredient (A) is 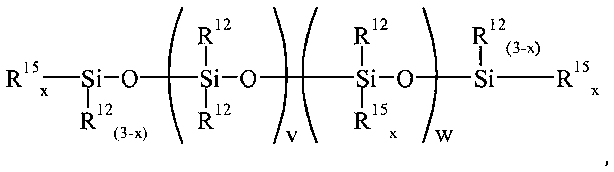 where each R¹² is independently a monovalent hydrocarbon group;
each R¹⁵ is independently an epoxy functional organic group;
each subscript x is independently 0 or 1;
subscript v has a value ranging from 0 to 10,000; and
subscript w has a value ranging from 0 to 10,000 or
where the n-alkyl glucamine is n-methyl glucamine.

7. The method of claim 5, further comprising preparing the epoxy functional polyorganosiloxane by hydrosilylation of ingredients comprising an alkenyl functional epoxy containing compound and a polyorganohydrogensiloxane.

8. The method of claim 7, where the alkenyl functional epoxy containing compound is allyl glycidyl ether, dodecyl glycidyl ether, tetradecyl glycidyl ether, or octadecylglycidyl ether or
where the ingredients further comprise an alkene, preferably where the alkene comprises undecene.

9. The method of claim 1, where step i) is performed by a method comprising:
1) reacting (a) an n-alkyl-glucamine with (b) an alkenyl functional epoxy compound, and
2) hydrosilylating the product of step 1) with (c) a polyorganohydrogensiloxane.

10. The method of claim 9,
where the n-alkyl glucamine is n-methyl glucamine or
where the alkenyl functional epoxy containing compound is allyl glycidyl ether, dodecyl glycidyl ether, tetradecyl glycidyl ether, or octadecylglycidyl ether.

11. The method of claim 1, where the oil is a silicone oil, preferably a polydialkylsiloxane.

12. A method for preparing an emulsion comprising
I) adding an aqueous phase to the product prepared by the method of any one of claims 1 to 11, and
II) mixing.

13. The method of claim 12,
where the method further comprises step III) subjecting the emulsion to shear during and/or after step II) or
where step I) and step II) are performed incrementally by adding a portion of the aqueous phase, mixing, and thereafter repeating until all of the aqueous phase is added or
where step I) and step II) are performed by adding the aqueous phase continuously over a period of time while mixing is performed or
further comprising adding a second oil during step I) or
where the aqueous phase is present in an amount ranging from 20 % to 95 % by weight based on the weight of the emulsion or
where the aqueous phase comprises water and an additional ingredient selected from salt and glycerol and a combination thereof.

14. A composition comprising:
a) an emulsion prepared by the method of any one of claims 12 to 13; and
b) an additional ingredient.

15. The composition of claim 14,
where ingredient (b) is selected from: additional silicones, aerosols, anti-oxidants, cleansing agents, colorants, additional conditioning agents, deposition agents, electrolytes, emollients and oils, exfoliating agents, foam boosters, fragrances, humectants, occlusive agents, pediculicides, pH control agents, pigments, preservatives, biocides, other solvents, stabilizers, sunscreening agents, suspending agents, tanning agents, other surfactants, thickeners, vitamins, botanicals, waxes, rheology-modifying agents, antiperspirants, anti-dandruff, anti-acne, anti-carie and wound healing-promotion agents, an additional oil, a hydrophilic medium, a filler, a fiber, a film forming polymer, an additional surfactant and/or emulsifier, a dyestuff, a structuring agent, an active ingredient, a fragrance, a preservative, and combinations thereof or
where the composition is a personal care product selected from antiperspirants and deodorants, skin creams, skin care lotions, moisturizers, facial treatments such as acne or wrinkle removers, personal and facial cleansers, bath oils, perfumes, colognes, sachets, sunscreens, pre-shave and after-shave lotions, shaving soaps, and shaving lathers, hair shampoos, hair conditioners, hair colorants, hair relaxants, hair sprays, mousses, gels, permanents, depilatories, and cuticle coats, make-ups, color cosmetics, foundations, concealers, blushes, lipsticks, eyeliners, mascara, oil removers, color cosmetic removers, wrinkle fillers, skin imperfection hiders, skin surface smoothers, eyelash curlers, nail varnishes, hair make-up products, eye shadows, body makeups, and powders, medicament creams, pastes or sprays including anti-acne, dental hygienic, antibiotic, healing promotive, and nutritive, which may be preventative and/or therapeutic.

## Patentansprüche

1. Verfahren, umfassend:
i) Herstellen eines Saccharid-Siloxan-Copolymers in Gegenwart eines Lösungsmittels, wobei das Saccharid-Siloxan-Copolymer folgende Formel aufweist
R²ₐR¹₍₃₋ₐ₎SiO-[(R²R¹SiO)ⱼ(R¹₂SiO)ₖ]_{y}-SiR¹₍₃₋ₐ₎R²ₐ;
wobei
jeder Index a unabhängig 0, 1, 2 oder 3 ist;
jeder Index j unabhängig eine ganze Zahl mit einem Wert im Bereich von 0 bis 10.000 ist;
jeder Index k unabhängig eine ganze Zahl mit einem Wert im Bereich von 0 bis 10.000 ist;
Index y eine solche ganze Zahl ist, dass das Copolymer ein Molekulargewicht bis zu 1.000.000 aufweist;
jedes R¹ gleich oder unterschiedlich sein kann und Wasserstoff, eine Alkylgruppe, ein organisches Radikal oder eine Gruppe der Formel R³-Q umfasst;
Q eine Epoxid-, Cycloalkylepoxid-, primäre oder sekundäre Amino-, Ethylendiamin-, Carboxy-, Halogen-, Vinyl-, Allyl-, Anhydrid- oder Mercapto-Funktionalität umfasst;
jedes R² die Formel Z-(G¹)ₙ-(G²)ₒ aufweist und es durchschnittlich mindestens ein R² pro Polymermolekül gibt;
G¹ ein Saccharidbestandteil ist, der 5 bis 12 Kohlenstoffatome umfasst;
eine Menge (n + o) 1 bis 10 ist und
Index n oder Index o 0 sein kann;
G² ein Saccharidbestandteil ist, der 5 bis 12 Kohlenstoffatome umfasst, und
G² zusätzlich mit einem organischen Radikal oder einem Organosiliciumradikal substituiert ist;
jedes Z eine Verknüpfungsgruppe ist, die unabhängig ausgewählt ist aus der Gruppe bestehend aus
-R³-NHC(O)-R⁴-;
-R³-NHC(O)O-R⁴-;
-R³-NH-C(O)-NH-R⁴-;
-R³-C(O)-O-R⁴-;
-R³-O-R⁴-;
-R³-CH(OH)-CH₂-O-R⁴-;
-R³-S-R⁴-;
-R³-CH(OH)-CH₂-NH-R⁴-;
-R³-N(R¹)-R⁴-;
-NHC(O)-R⁴-;
-NHC(O)O-R⁴-;
-NH-C(O)-NH-R⁴-;
-C(O)-O-R⁴-;
-O-R4-;
-CH(OH)-CH₂-O-R⁴-;
-S-R⁴-;
-CH(OH)-CH₂-NH-R⁴-;
-N(R¹)-R⁴-;
-R³-NHC(O)-;
-R³-NHC(O)O-;
-R³-NH-C(O)-NH-;
-R³-C(O)-O-;
-R³-O-;
-R³-CH (OH)-CH₂-O-;
-R³-S-;
-R³-CH(OH)-CH₂-NH-; und
-R³-N(R¹)-;
wobei jedes R³ und jedes R⁴ unabhängig eine zweiwertige Abstandsgruppe ist, die eine Gruppe der Formel (R⁵)r(R⁶)s(R⁷)t umfasst,
wobei mindestens einer der Indizes r, s und t 1 ist;
jedes R⁵ und jedes R⁷ unabhängig entweder eine Alkylgruppe mit 1 bis 12 Kohlenstoffatomen oder eine Gruppe der Formel (R⁹O)p ist, wobei R⁹ eine Kohlenwasserstoffgruppe mit 1 bis 12 Kohlenstoffatomen ist, Index p eine ganze Zahl im Bereich von 1 bis 50 ist und jedes R⁹O gleich oder unterschiedlich sein kann;
R⁶ -N(R⁸)- ist, wobei R⁸ Wasserstoff, eine Alkylgruppe mit 1 bis 12 Kohlenstoffatomen, eine Gruppe der Formel Z-X oder R³ ist und
jedes X unabhängig ein Carbonsäure-, Phosphat-, Sulfat-, Sulfonat- oder quartärer Ammoniumradikal sein kann;
wahlweise ii) Entfernen eines Teils des Lösungsmittels; und iii) Hinzufügen eines Öls,
wobei das Öl vor und/oder während Schritt i) zugegeben wird.

2. Verfahren nach Anspruch 1,
wobei jeder Index j unabhängig eine ganze Zahl mit einem Wert im Bereich von 0 bis 500 ist und jeder Index k unabhängig eine ganze Zahl mit einem Wert im Bereich von 0 bis 500 ist oder wobei jedes R⁵ und jedes R⁷ unabhängig eine Alkylgruppe mit 1 bis 12 Kohlenstoffatomen ist, oder
ferner umfassend: iv) Entfernen des Lösungsmittels oder wobei Schritt i) durch Umsetzen von Inhaltsstoffen durchgeführt wird, die Folgendes umfassen:
(A) ein aminofunktionelles Polyorganosiloxan und
(B) ein Zuckerlacton oder
wobei das Produkt von Schritt i) sekundäre Aminfunktionalität enthält, ferner umfassend einen Schritt des Umsetzens des Produkts von Schritt i) mit einem Verkappungsmittel, ausgewählt aus einem Lacton, einer halogenierten ungesättigten Verbindung, einer epoxidfunktionellen Verbindung oder einem Säureanhydrid, oder wobei das Lösungsmittel ein Alkohol ist, ausgewählt aus Methanol, Ethanol, n-Propanol, Isopropanol, 2-Propanol, Isobutanol, n-Butanol und Kombinationen davon, oder wobei das Öl ein organisches Öl ist, ausgewählt aus einem Kohlenwasserstofföl, einem Ester, einem Pflanzenöl, einem Mineralöl oder einem Fettalkohol, oder wobei das Copolymer und das Öl in solchen Mengen vorliegt, dass das Gewichtsverhältnis von Copolymer/Öl im Bereich von 1/1 bis 1/50 liegt.

3. Verfahren nach Anspruch 1, wobei Inhaltsstoff (A) Folgendes ist 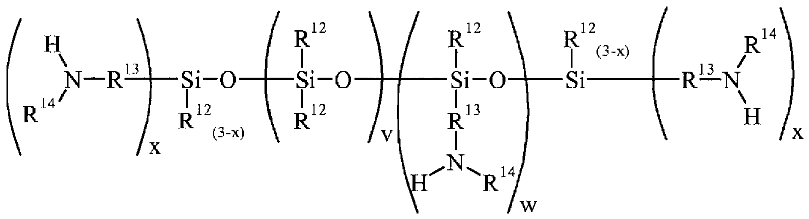 wobei
jedes R¹² unabhängig eine einwertige Kohlenwasserstoffgruppe ist;
jedes R¹³ unabhängig eine zweiwertige organische Gruppe ist;
jedes R¹⁴ unabhängig ein Wasserstoffatom oder eine einwertige Kohlenwasserstoffgruppe mit 1 bis 4 Kohlenstoffatomen ist;
jeder Index x unabhängig 0 oder 1 ist;
Index v einen Wert im Bereich von 0 bis 10.000 aufweist; und
Index w einen Wert im Bereich von 0 bis 10.000 aufweist und wobei vorzugsweise
Inhaltsstoff (A) aus mit Trimethylsiloxy beendetem Poly(dimethylsiloxan/
Methyl(aminoethylaminoisobutyl)siloxan), mit Trimethylsiloxy beendetem Poly(dimethylsiloxan/Methyl(aminopropyl)siloxan), mit Trimethylsiloxy beendetem Poly(dimethylsiloxan/Methyl(aminoethylaminopropyl)siloxan) und Kombinationen davon ausgewählt ist.

4. Verfahren nach Anspruch 1, wobei Inhaltsstoff (B) 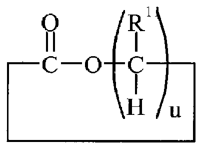 ist, wobei jedes R¹¹ unabhängig ein Wasserstoffatom, eine Hydroxylgruppe, eine Alkoxygruppe oder eine Saccharidgruppe ist; und
Index u einen Wert im Bereich von 5 bis 12 aufweist und wobei vorzugsweise
Inhaltsstoff (B) aus Butyrolacton, epsilon-Caprolacton und delta-Gluconolacton ausgewählt ist.

5. Verfahren nach Anspruch 1, wobei Schritt i) durch Umsetzen von Inhaltsstoffen durchgeführt wird, die Folgendes umfassen:
(A) ein epoxyfunktionelles Polyorganosiloxan und
(B) ein n-Alkylglucamin

6. Verfahren nach Anspruch 5, wobei Inhaltsstoff (A) Folgendes ist 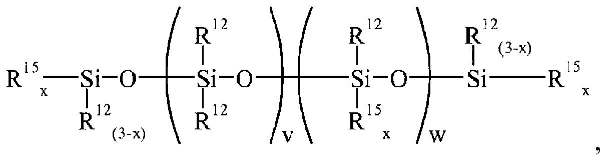 wobei jedes R¹² unabhängig eine einwertige Kohlenwasserstoffgruppe ist; jedes R¹⁵ unabhängig eine epoxyfunktionelle organische Gruppe ist; jeder Index x unabhängig 0 oder 1 ist;
Index v einen Wert im Bereich von 0 bis 10.000 aufweist; und
Index w einen Wert im Bereich von 0 bis 10.000 aufweist oder wobei das n-Alkylglucamin n-Methylglucamin ist.

7. Verfahren nach Anspruch 5, ferner umfassend: Herstellen des epoxyfunktionellen Polyorganosiloxans durch Hydrosilylierung von Inhaltsstoffen, die eine alkenylfunktionelle epoxyhaltige Verbindung und ein Polyorganohydrogensiloxan umfassen.

8. Verfahren nach Anspruch 7, wobei die alkenylfunktionelle epoxyhaltige Verbindung Allylglycidylether, Dodecylglycidylether, Tetradecylglycidylether oder Octadecylglycidylether ist oder
wobei die Inhaltsstoffe ferner ein Alken umfassen, wobei vorzugsweise das Alken Undecen umfasst.

9. Verfahren nach Anspruch 1, wobei Schritt i) durch ein Verfahren durchgeführt wird, das Folgendes umfasst:
1) Umsetzen (a) eines n-Alkylglucamins mit (b) einer alkenylfunktionellen Epoxyverbindung und
2) Hydrosilylieren des Produkts von Schritt 1) mit (c) einem Polyorganohydrogensiloxan.

10. Verfahren nach Anspruch 9,
wobei das n-Alkylglucamin n-Methylglucamin ist oder
wobei die alkenylfunktionelle epoxyhaltige Verbindung Allylglycidylether, Dodecylglycidylether, Tetradecylglycidylether oder Octadecylglycidylether ist.

11. Verfahren nach Anspruch 1, wobei das Öl ein Silikonöl, vorzugsweise ein Polydialkylsiloxan ist.

12. Verfahren zum Herstellen einer Emulsion, umfassend
I) Zugeben einer Wasserphase zu dem Produkt, das mit dem Verfahren nach einem der Ansprüche 1 bis 11 hergestellt wurde, und
II) Mischen.

13. Verfahren nach Anspruch 12,
wobei das Verfahren ferner umfasst: Schritt III) Anwenden von Scherung auf die Emulsion während und/oder nach Schritt II) oder wobei Schritt I) und Schritt II) inkrementell durch Zugabe eines Teils der Wasserphase, Mischen und danach Wiederholen, bis die gesamte Wasserphase zugegeben ist, durchgeführt werden oder
wobei Schritt I) und Schritt II) durch kontinuierliches Zugeben der Wasserphase über einen Zeitraum unter Mischen durchgeführt werden oder
ferner das Zugeben eines zweiten Öls während Schritt I) umfassend oder wobei die Wasserphase in einer Menge im Bereich von 20 Gew.-% bis 95 Gew.-%, bezogen auf das Gewicht der Emulsion, vorhanden ist oder
wobei die Wasserphase Wasser und einen zusätzlichen Inhaltsstoff, ausgewählt aus Salz und Glycerol und einer Kombination davon, umfasst.

14. Zusammensetzung, umfassend:
a) eine Emulsion, hergestellt mit dem Verfahren nach einem der Ansprüche 12 bis 13; und
b) einen zusätzlichen Inhaltsstoff.

15. Zusammensetzung nach Anspruch 14,
wobei Inhaltsstoff (b) ausgewählt ist aus: zusätzlichen Silikonen, Aerosolen, Antioxidationsmitteln, Reinigungsmitteln, Farbstoffen, zusätzlichen Konditioniermitteln, Anlagerungsmitteln, Elektrolyten, Weichmachern und Ölen, Peelingmitteln, Schaumverstärkern, Duftstoffen, Feuchthaltemitteln, porenverschließenden Mitteln, Pedikuloziden, pH-Steuermitteln, Pigmenten, Konservierungsstoffen, Bioziden, anderen Lösungsmitteln, Stabilisierungsmitteln, Sonnenschutzmitteln, Suspendiermitteln, Bräunungsmitteln, anderen Tensiden, Verdickungsmitteln, Vitaminen, pflanzlichen Mitteln, Wachsen, Rheologiemodifizierungsmitteln, schweißhemmenden Mitteln, Antischuppenmitteln, aknehemmenden Mitteln, Antikariesmitteln und Wundheilungsförderungsmitteln, einem zusätzlichen Öl, einem hydrophilen Mittel, einem Füllmittel, einer Faser, einem filmbildenden Polymer, einem zusätzlichen Tensid und/oder Emulgator, einem Farbstoff, einem Strukturbildner, einem Wirkstoffbestandteil, einem Parfüm, einem Konservierungsstoff und Kombinationen davon, oder wobei die Zusammensetzung ein Körperpflegeprodukt ist, ausgewählt aus Schweißhemmern und Deodorants, Hautcremes, Hautpflegelotionen, Feuchtigkeitscremes, Gesichtsbehandlungen, wie Akne- oder Faltenentfernern, Körper- und Gesichtsreinigern, Badeölen, Parfüms, Eaux de Cologne, Duftkissen, Sonnenschutzmitteln, Preshave- und Aftershavelotionen, Rasierseifen und Rasierschäumen, Haarshampoos, Haarkonditioniermitteln, Haarfärbemitteln, Haarentspannungsmitteln, Haarsprays, -schäumen, -gelen, Dauerwellen, Enthaarungsmitteln und Kutikulabeschichtungen, Make-ups, Farbkosmetika, Grundierungen, Concealern, Rouge, Lippenstiften, Kayalstiften, Wimperntusche, Abschminkölen, Farbkosmetikentfernern, Faltenfüllern, Abdeckmitteln für Hautfehler, Hautoberflächenglättungsmitteln, Wimpernformern, Nagellacken, Haar-Make-up-Produkten, Lidschatten, Körper-Make-ups und -Puder, medizinische Cremes, Pasten oder Sprays, einschließlich solchen, die aknehemmend, dentalhygienisch, antibiotisch, heilungsfördernd und nährend sind, die präventativ und/oder therapeutisch sein können.

## Revendications

1. Procédé comprenant :
i) la préparation d'un copolymère saccharide siloxane en présence d'un solvant, où le copolymère saccharide siloxane a la formule
R²ₐR¹(3-a)SiO-[(R²R¹SiO)ⱼ(R¹₂SiO)k]y-SiR¹(3-a)R²a,
dans laquelle
chaque indice a est indépendamment 0, 1, 2, ou 3;
chaque indice j est indépendamment un entier avec une valeur allant de 0 à 10 000 ; chaque indice k est indépendamment un entier avec une valeur allant de 0 à 10 000 ; l'indice y est un entier tel que le copolymère a une masse moléculaire jusqu'à 1 000 000 ; chaque R¹ peut être identique ou différent et comprend de l'hydrogène, un groupe alkyle, un radical organique, ou un groupe de formule R³-Q ;
Q comprend une fonctionnalité époxy, cycloalkyépoxy, amino primaire ou secondaire, éthylène-diamine, carboxy, halogène, vinyle, allyle, anhydride ou mercapto ;
chaque R² est de formule Z-(G¹)ₙ-(G²)ₒ, et il y a une moyenne d'au moins un R² par molécule de polymère ;
G¹ est un composant saccharide comprenant 5 à 12 atomes de carbone ;
une quantité (n + o) va de 1 à 10, et
l'indice n ou l'indice o peut être 0 ;
G² est un composant saccharide comprenant 5 à 12 atomes de carbone, et
G² est en outre substitué par un radical organique ou un radical organosilicié ;
chaque Z est un groupe de liaison indépendamment choisi dans le groupe constitué de
-R³-NHC(O)-R⁴- ;
-R³-NHC(O)O-R⁴- ;
-R³-NH-C(O)-NH-R⁴-;
-R³-C(O)-O-R⁴-;
-R³-O-R⁴-;
-R³-CH(OH)-CH₂-O-R⁴-;
-R³-S-R⁴-;
-R³-CH(OH)-CH₂-NH-R⁴- ;
-R³-N(R¹)-R⁴-;
-NHC(O)-R⁴-;
-NHC(O)O-R⁴-;
-NH-C(O)-NH-R⁴-;
-C(O)-O-R⁴-;
-O-R⁴-;
-CH(OH)-CH₂-O-R⁴-;
-S-R⁴-;
-CH(OH)-CH₂-NH-R⁴-;
-N(R¹)-R⁴-;
-R³-NHC(O)-;
-R³-NHC(O)O-;
-R³-NH-C(O)-NH-;
-R³-C(O)-O-;
-R³-O-;
-R³-CH(OH)-CH₂-O-;
-R³-S-;
-R³-CH(OH)-CH₂-NH-; et
-R³-N(R¹)-;
où chaque R³ et chaque R⁴ sont indépendamment un segment espaceur divalent comprenant un groupe de formule (R⁵)r(R⁶)s(R⁷)t,
où au moins l'un des indices r, s et t est 1 ;
chaque R⁵ et chaque R⁷ sont indépendamment ou un groupe alkyle de 1 à 12 atomes de carbone ou un groupe de formule (R⁹O)p, où R⁹ est un groupe hydrocarbure de 1 à 12 atomes de carbone, l'indice p est un nombre entier allant de 1 à 50, et chaque R⁹O peut être identique ou différent ;
R⁶ est -N(R⁸)-, où R⁸ est un hydrogène, un groupe alkyle de 1 à 12 atomes de carbone, un groupe de formule Z-X, ou R³ ; et
chaque X est indépendamment un radical acide carboxylique, phosphate, sulfate, sulfonate ou ammonium quaternaire ;
facultativement ii) l'élimination d'une partie du solvant ; et
iii) l'ajout d'une huile,
dans lequel l'huile est ajoutée avant et/ou pendant l'étape i).

2. Procédé selon la revendication 1,
où chaque indice j est indépendamment un entier avec une valeur allant de 0 à 500 et chaque indice k est indépendamment un entier avec une valeur allant de 0 à 500 ou où chaque R⁵ et chaque R⁷ sont indépendamment un groupe alkyle de 1 à 12 atomes de carbone ou
comprenant en outre iv) l'élimination du solvant ou
où l'étape i) est effectuée en faisant réagir des ingrédients comprenant :
(A) un polyorganosiloxane à fonction amino, et
(B) une lactone glucidique ou
où le produit de l'étape i) contient une fonctionnalité amine secondaire, comprenant en outre une étape consistant à faire réagir le produit de l'étape i) avec un agent de coiffage choisi parmi une lactone, un composé insaturé halogéné, un composé à fonction époxy, ou un anhydride acide ou
où le solvant est un alcool choisi parmi le méthanol, l'éthanol, le n-propanol, l'isopropanol, le 2-propanol, l'isobutanol, le n-butanol, et leurs combinaisons ou où l'huile est une huile organique choisie parmi une huile hydrocarbure, un ester, une huile végétale, une huile minérale, ou un alcool gras ou
où le copolymère et l'huile sont présents en des quantités telles qu'un rapport pondéral de copolymère/huile va de 1/1 à 1/50.

3. Procédé selon la revendication 1, où l'ingrédient (A) est 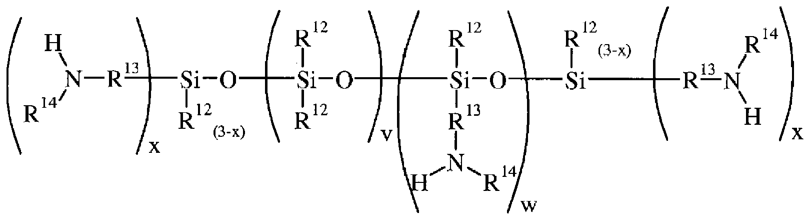 où
chaque R¹² est indépendamment un groupe hydrocarbure monovalent ;
chaque R¹³ est indépendamment un groupe organique divalent ;
chaque R¹⁴ est indépendamment un atome d'hydrogène ou un groupe hydrocarbure monovalent de 1 à 4 atomes de carbone ;
chaque indice x est indépendamment 0 ou 1 ;
l'indice v a une valeur allant de 0 à 10 000 ; et
l'indice w a une valeur allant de 0 à 10 000 et de préférence
où l'ingrédient (A) est choisi parmi un poly(diméthylsiloxane/méthyl(aminoéthylaminoisobutyl)siloxane) à terminaison triméthylsiloxy, un poly(diméthylsiloxane/méthyl(aminopropyl)siloxane) à terminaison triméthylsiloxy, un poly(diméthylsiloxane/méthyl(aminoéthylaminopropyl)siloxane) à terminaison triméthylsiloxy, et leurs combinaisons.

4. Procédé selon la revendication 1, où l'ingrédient (B) est 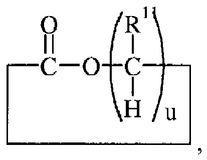 où chaque R¹¹ est indépendamment un atome d'hydrogène, un groupe hydroxyle, un groupe alcoxy, ou un groupe saccharide ; et
l'indice u a une valeur allant de 5 à 12 et de préférence
où l'ingrédient (B) est choisi parmi la butyrolactone, l'epsilon caprolactone et la delta gluconolactone.

5. Procédé selon la revendication 1, où l'étape i) est effectuée en faisant réagir des ingrédients comprenant :
(A) un polyorganosiloxane à fonction époxy, et
(B) une n-alkyl glucamine

6. Procédé selon la revendication 5, où l'ingrédient (A) est 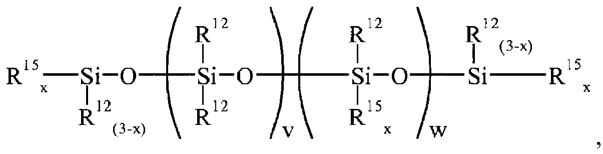 où
chaque R¹² est indépendamment un groupe hydrocarbure monovalent ;
chaque R¹⁵ est indépendamment un groupe organique à fonction époxy ;
chaque indice x est indépendamment 0 ou 1 ;
l'indice v a une valeur allant de 0 à 10 000 ; et
l'indice w a une valeur allant de 0 à 10 000 ou
où la n-alkyl glucamine est la n-méthyl glucamine.

7. Procédé selon la revendication 5, comprenant en outre la préparation du polyorganosiloxane à fonction époxy par hydrosilylation d'ingrédients comprenant un composé contenant un époxy à fonction alcényle et un polyorganohydrogénosiloxane.

8. Procédé selon la revendication 7, où le composé contenant un époxy à fonction alcényle est un éther allylglycidylique, un éther dodécylglycidylique, un éther tétradécylglycidylique, ou un éther octadécylglycidylique ou
où les ingrédients comprennent en outre un alcène, de préférence où l'alcène comprend de l'undécène.

9. Procédé selon la revendication 1, où l'étape i) est effectuée par un procédé comprenant :
1) la réaction (a) d'une n-alkyl-glucamine avec (b) un composé époxy à fonction alcényle, et
2) l'hydrosilylation du produit de l'étape 1) avec (c) un polyorganohydrogénosiloxane.

10. Procédé selon la revendication 9,
où la n-alkyl glucamine est la n-méthyl glucamine ou
où le composé contenant un époxy à fonction alcényle est un éther allylglycidylique, un éther dodécylglycidylique, un éther tétradécylglycidylique, ou un éther octadécylglycidylique ou

11. Procédé selon la revendication 1, où l'huile est une huile de silicone, de préférence un polydialkylsiloxane.

12. Procédé de préparation d'une émulsion comprenant
I) l'ajout d'une phase aqueuse au produit préparé par le procédé selon l'une quelconque des revendications 1 à 11, et
II) le mélange.

13. Procédé selon la revendication 12,
où le procédé comprend en outre l'étape III) consistant à soumettre l'émulsion à un cisaillement durant et/ou après l'étape II) ou
où l'étape I) et l'étape II) sont exécutées de manière incrémentielle en ajoutant une partie de la phase aqueuse, en mélangeant, puis en répétant jusqu'à ce que la totalité de la phase aqueuse soit ajoutée ou
où l'étape I) et l'étape II) sont effectuées en ajoutant la phase aqueuse de façon continue sur une période de temps tandis que le mélange est effectué ou
comprenant en outre l'ajout d'une deuxième huile pendant l'étape I) ou
où la phase aqueuse est présente en une quantité allant de 20 % à 95 % en poids sur base du poids de l'émulsion ou
où la phase aqueuse comprend de l'eau et un ingrédient additionnel choisi parmi un sel et du glycérol et une combinaison de ceux-ci.

14. Composition comprenant :
a) une émulsion préparée par le procédé selon l'une quelconque des revendications 12 à 13 ; et
b) un ingrédient additionnel.

15. Composition selon la revendication 14,
où l'ingrédient (b) est choisi parmi : des silicones supplémentaires, des aérosols, des antioxydants, des agents nettoyants, des colorants, des agents de conditionnement supplémentaires, des agents de dépôt, des électrolytes, des émollients et huiles, des agents exfoliants, des agents moussants, des parfums, des humectants, des agents occlusifs, des pédiculicides, des agents de contrôle du pH, des pigments, des conservateurs, des biocides, d'autres solvants, des agents stabilisants, des agents d'écran solaire, des agents de suspension, des agents bronzants, d'autres agents tensioactifs, des épaississants, des vitamines, des produits végétaux, des cires, des agents modifiant la rhéologie, des antitranspirants, des agents anti-pelliculaires, anti-acnéiques, anti-caries et favorisant la cicatrisation, une huile supplémentaire, un milieu hydrophile, une charge, une fibre, un polymère filmogène, un agent tensioactif et/ou émulsifiant supplémentaire, un colorant, un agent structurant, un ingrédient actif, un parfum, un conservateur, et leurs combinaisons ou où la composition est un produit de soins d'hygiène personnelle choisi parmi des antitranspirants et déodorants, des crèmes pour la peau, des lotions de soin de la peau, des agents hydratants, des traitements du visage tels que des produits contre l'acné ou les rides, des nettoyants personnels et pour le visage, des huiles de bain, des parfums, des eaux de Cologne, des sachets, des écrans solaires, des lotions avant-rasage et après-rasage, des savons de rasage, et des mousses à raser, des shampooings pour les cheveux, des après-shampooings, des teintures capillaires, des relaxants pour cheveux, des aérosols pour cheveux, des mousses, des gels, des permanentes, des dépilatoires, et revêtements de cuticule, des maquillages, des cosmétiques de couleur, des fonds de teint, des correcteurs, des fards à joues, des rouges à lèvres, des crayons pour les sourcils, un mascara, des produits pour éliminer l'huile, des produits pour éliminer les cosmétiques de couleur, des fluides anti-rides, des masquants pour imperfections de la peau, des lisseurs de surface de la peau, des boucleurs de cils, des vernis à ongles, des produits de maquillage pour cheveux, des ombres à paupières, des maquillages pour le corps, et poudres, des crèmes, pâtes ou aérosols médicamenteux y compris anti-acnéiques, d'hygiène dentaire, antibiotiques, favorisant la guérison, et nutritifs qui peuvent être préventifs et/ou thérapeutiques.
